(19) Europäisches Patentamt
European Patent Office
Office européen des brevets

(11) Veröffentlichungsnummer: **0 165 608**
**B1**

(12) **EUROPÄISCHE PATENTSCHRIFT**

(45) Veröffentlichungstag der Patentschrift: **02.01.91**

(21) Anmeldenummer: **85107628.1**

(22) Anmeldetag: **20.06.85**

(51) Int. Cl.^5: **C 07 D 251/24,** C 07 D 251/22, C 07 D 401/04, C 07 D 413/12, C 07 D 417/12, G 03 C 1/815

# (54) Hydroxyphenyltriazine, Verfahren zu ihrer Herstellung und ihre Verwendung als UV-Absorber.

(30) Priorität: **22.06.84 CH 3028/84**

(43) Veröffentlichungstag der Anmeldung: **27.12.85 Patentblatt 85/52**

(45) Bekanntmachung des Hinweises auf die Patenterteilung: **02.01.91 Patentblatt 91/01**

(84) Benannte Vertragsstaaten: **BE CH DE FR GB IT LI NL**

(56) Entgegenhaltungen:
**GB-A-1 216 389**
**US-A-3 278 534**
**US-A-3 444 164**

(73) Patentinhaber: **ILFORD AG**
**Industriestrasse 15**
**CH-1701 Fribourg (CH)**

(72) Erfinder: **Fryberg, Mario, Dr.**
**Derry-le-Mont**
**CH-1724 Praroman-Le-Mouret (CH)**
Erfinder: **Jan, Gérald, Dr.**
**Rte. de la Poudrière 33**
**CH-1700 Fribourg (CH)**
Erfinder: **Mariaca, Raul, Dr.**
**Au Village 45**
**CH-1751 Autigny (CH)**
Erfinder: **Kramp, Ekkehard, Dr.**
**Route de Planafin 49**
**CH-1723 Marly (CH)**

(74) Vertreter: **Sandmair, Kurt, Dr. et al**
**Patentanwälte Schwabe, Sandmair, Marx**
**Stuntzstrasse 16**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 165 608 B1

**Beschreibung**

Die vorliegende Erfindung betrifft Hydroxyphenyltriazine, Verfahren zu ihrer Herstellung und ihre Verwendung als UV-Absorber.

Hydroxyphenyltriazine, die z.B. der Formel

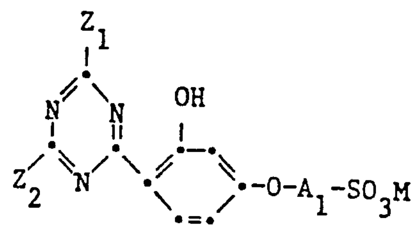

entsprechen, worin $A_1$ Alkylen, M z.B. Wasserstoff oder eine Alkalimetall ist, und $Z_1$ und $Z_2$ z.B. den Rest der Formel

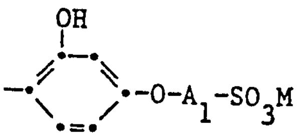

bedeuten, sind aus US 3.444.164 bekannt. Sie werden durch umsetzung von Verbindungen der Formel

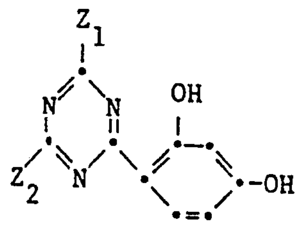

mit Sultonen der Formel

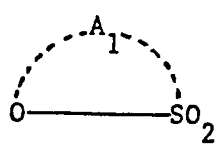

hergestellt. Aufgrund ihres Absorptionsvermögens von UV-Licht eignen sich sich als Lichtschutzmittel für organische Materialien wie z.B. für natürliche oder synthetische Polymere.

So können sie beispielsweise auch in Gelatinescichten photograpischer Materialien als UV-Absorber verwendet werden.

Die Verbindungen gemäss US 3.444.164 besitzen im Hinblick auf die Verwendung in photographischen Materialien eine Reihe von Nachteilen. Vor allem ist die geringe Wasserlöslichkeit und die starke Viskositäserhöhung zu nennen, die sie bewirken, wenn sie wässrigen Gelatinelösungen hinzugegeben werden.

Aufgabe der vorliegenden Erfindung ist deshalb die Bereitstellung neuer UV-Absorber vom Hydroxyphenyltriazintyp mit verbesserten Applikationseigenschaften.

Es wurde nun gefunden, dass die Einführung einer zweiten Hydroxylgruppe in den Phenylsubstituenten des Triazinringes zu Verbindungen führt, die sich sowohl durch eine bemerkenswerte Erhöhung der Wasserlöslichkeit als auch eine wesentlich geringere Beeinflussung der Viskosität von wässrigen Gelatinelösungen auszeichnen.

Gegenstand der vorliegenden Erfindung sind somit Verbindungen der Formel

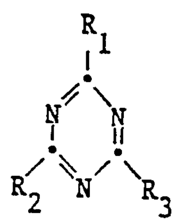

(1)

worin mindestens einer der Substituenten $R_1$, $R_2$ und $R_3$ ein Rest der Formel

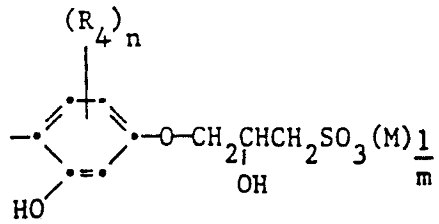

ist, worin M ein Alkali- oder Erdalkalimetall, Ammonium oder Tetraalkylammonium, worin die Alkylreste unabhängig voneinander 1 bis 4 Kohlenstoffatome enthalten, m 1 oder 2, $R_4$ Alkyl oder Alkoxy mit je 1 bis 4 Kohlenstoffatomen, Hydroxyl oder einen Rest der Formel —COR, worin R Alkyl mit 1 bis 8 Kohlenstoffatomen oder Phenyl bedeutet, und n 0, 1, 2 oder 3 ist, und der übrige Substituent bzw. die übrigen Substituenten unabhängig voneinander Alkyl, mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2H(OH)CH_2SO_3K$, Cyclopentyl, Cyclohexyl, Phenyl, Hydroxyl, Halogen, (Di-)-Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl ist bzw. sind, wobei dieser Substituent bzw. diese Substituenten auch über ein Sauerstoff-, Schwefel- oder Stickstoffatom an den Triazinylrest gebunden sein kann bzw. sein können, wobei ein über ein Stickstoffatom an den Triazinylring gebundener Phenylrest zusätzlich noch mit Thiazol-, Benzthiazol-, Oxazol-, Isoxazol-, und Benzoxazolgruppen substituiert sein kann, oder dieser Substituent bzw. diese Substituenten ein stickstoffhaltiger Heterocyclus mit 3 bis 6 Ringatomen ist bzw. sind.

Weitere Gegenstände der vorliegenden Erfindung sind ein Verfahren zur Herstellung der Verbindung der Formel (1), die Verwendung dieser Verbindungen als UV-Absorber in organischen Materialien, insbesondere photographischen Materialien, und die die erfindungsgemässen Verbindungen enthaltenden photographischen Materialien.

In den Verbindungen der Formel (1) bedeutet mindestens einer der Substituenten $R_1$, $R_2$ und $R_3$ einen Rest der Formel

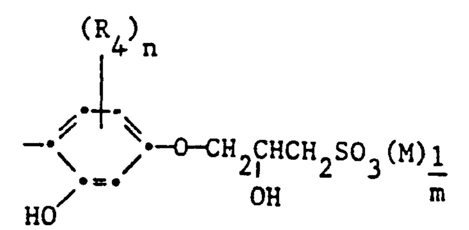

Hierin ist M ein Alkali- oder Erdalkalimetall, wie z.B. Natrium, Kalium, Magnesium und Calcium, oder Ammonium oder auch Tetraalkylammonium, worin die Alkylreste unabhängig voneinander 1 bis 4 Kohlenstoffatome enthalten. Beispielsweise kommen Butyl, Propyl, Aethyl und insbesondere Methyl in Frage. Vorzugsweise ist M Natrium oder Kalium. Der Index m bedeutet 1 oder 2 und gibt die Wertigkeit von M an.

$R_1$, $R_2$ und $R_3$ können ferner Alkyl oder Aryl bedeuten. Unter Alkyl und Aryl soll sowohl unsubstituiertes als auch substituiertes Alkyl und Aryl verstanden werden. Bevorzugte Alkylgruppen enthalten 1 bis 12 Kohlenstoffatome und sind Methyl, Aethyl, Propyl, Butyl, Pentyl, Hexyl, Heptyl, Octyl, Nonyl, Decyl, Undecyl oder Dodecyl oder Isomere dieser Reste. Besonders bevorzugte Alkylgruppen enthalten 1 bis 4 Kohlenstoffatome.

Geeignete Arylgruppen $R_1$, $R_2$ und $R_3$ sind z.B. Naphthyl und insbesondere Phenyl.

Diese Alkyl- und Arylgruppen sind entweder direkt (über eines ihrer Kohlenstoffatome) oder indirekt über ein Heteroatom an den Triazinylrest gebunden. Geeignete Heteratome sind z.B. Sauerstoff-, Schwefel- und Stickstoffatome, letztere insbesondere als Teil einer Imino- oder Alkyliminogruppe, die vorzugsweise 1 bis 4 Kohlenstoffatome im Alkylrest enthält. Der Triazinylrest in den Verbindungen der Formel (1) kann also neben den genannten Alkyl- und Arylgruppen z.B. auch Alkoxy-, Thioalkyl-, (Di-) Alkylamino- (d.h. Substituenten der Formel

$(C_1—C_{12}—Alkyl)N—(C_1—C_4\text{-}Alkyl)$, und $(C_1—C_{12}—Alkyl)N—H)$,

Phenoxy-, Thiophenyl- und Anilinosubstituenten (d.h. Substituenten der Formel

$C_6H_5—N—H$ oder $C_6H_5—N(C_1—C_4\text{-Alkyl}))$

enthalten, wenn diese Alkyl- und Arylgruppen über eines der genannten Heteroatome an den Triazinylrest gebunden sind.

Substituenten am Triazinylrest können somit Alkoxy-, Alkylamino- und Thioalkyl mit je 1 bis 12 Kohlenstoffatomen sowie Dialkylamino mit insgesamt 2 bis 16 Kohlenstoffatomen sein. Bevorzugte Alkoxy- und Alkylaminosubstituenten enthalten 1 bis 4 Kohlenstoffatome, besonders geeignete Thioalkylsubstituenten 8 bis 12 Kohlenstoffatome.

Die Reste $R_1$, $R_2$ und $R_3$ können weiter substituiert sein. Beispielsweise sind Methoxy, Hydroxyl, Phenyl, Piperidinyl, Pyrrolidinyl oder Carbalkoxy mit 2 bis 9 Kohlenstoffatomen die bevorzugten Substituenten an den Alkyl- bzw. Heteroatom-Alkylresten.

Für die Aryl- bzw. Heteroatom-Arylreste, insbesondere die Phenyl-, Phenoxy-, Thiophenyl- und Anilinoreste kommen z.B. Alkyl- oder Alkoxyreste mit je 1 bis 12 Kohlenstoffatomen, wie Methyl, tert.Butyl, Pentyl, Octyl, Decyl, Dodecyl, Methoxy, Butoxy oder Pentoxy, die mit Hydroxyl, Methoxy oder Carbalkoxy mit 2 bis 9 Kohlenstoffatomen substituiert sein können, oder substituierte Alkylreste wie $—CH_2CH(OH)CH_2SO_3K$, ferner Cyclopentyl, Cyclohexyl, Phenyl, Hydroxyl, Halogen, insbesondere Chlor, (Di-)-Alkylamino mit (je) 1 bis 4 Kohlenstoffatomen im Alkylrest, Nitro, Cyano, Carbalkoxy mit 2 bis 9 Kohlenstoffatomen und Sulfogruppen wie z.B. $—SO_3H$, $—SO_3Na$, $—SO_3K$ oder $—SO_3NH_4$ als Substituenten in Frage.

Speziell die Anilinoreste $R_1$ bis $R_3$ können heterocyclische Substituenten enthalten, wie z.B. Thiazol-, Benzthiazol-, Oxazol-, Isoxazol- und Benzoxazolgruppen. Bevorzugt sind Benzthiazolgruppen, insbesondere die der Formel

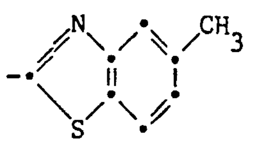

und Isoxazolgruppen, insbesondere die der Formel

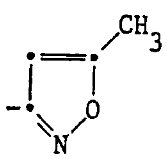

Ferner können $R_1$, $R_2$ und $R_3$ je einen Heterocyclus bedeuten. Insbesondere kommen stickstoffhaltige Heterocyclen in Frage, die vorzugsweise 3 bis 6 Ringatome enthalten, wobei eines ein zweites Heteroatom sein kann wie z.B. Sauerstoff. Diese Heterocyclen können gesättigt oder ungesättigt, substituiert oder unsubstituiert sein. Sie können sowohl über das eigene Heteroatom als auch ein Kohlenstoffatom an den Triazinylrest gebunden sein. Geeignete Beispiele sind die Reste der Formeln

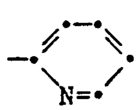 , 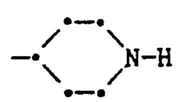 , 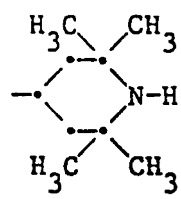 , 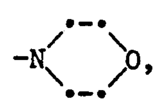 , 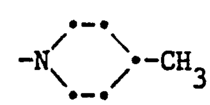 ,

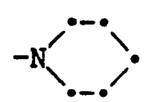 , 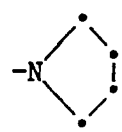 und 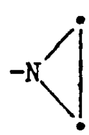 ,

Der Substituent $R_4$ ist Alkyl oder Alkoxy mit je 1 bis 4 Kohlenstoffatomen, also z.B. Butyl, tert. Butyl, Propyl, Aethyl, Aethoxy und vorzugsweise Methyl und Methoxy.

Ferner bedeutet $R_4$ Hydroxyl oder einen Rest der Formel —COR, worin R Alkyl mit 1 bis 8, vorzugsweise 1 bis 4 Kohlenstoffatomen, insbesondere Methyl, oder Phenyl bedeutet.

Der Index n ist 0, 1, 2 oder 3, wobei 0 und 1 bevorzugt sind.

Die Substituenten $R_1$, $R_2$ und $R_3$ können unabhängig voneinander die für sie aufgezählten Bedeutungen besitzen.

Bevorzugte Verbindungen der Formel (1) sind solche, worin $R_2$ und $R_3$ ein Rest der Formel

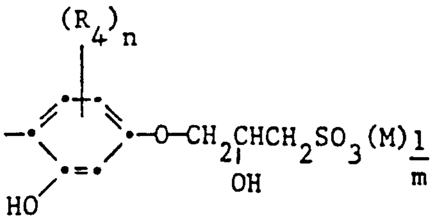

sind, worin $R_4$ Methyl, Methoxy, Hydroxyl oder Acetyl, n 1, 2 oder 3, vorzugsweise 1, ist, M und m die oben angegebene Bedeutung haben und $R_1$ Alkyl, mit 1 bis Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)-Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl, über ein Sauerstoff-, Schwefel- oder Stickstoffatom an den Triazinylrest gebundenes Alkyl mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl, oder ein stickstoffhaltiger Heterocyclus mit 3 bis 6 Ringatomen ist.

In weiteren besonders bevorzugten Verbindungen der Formel (1) ist $R_1$ Phenyl, Anilino oder N-Alkyl-Anilino ist, wobei diese Reste mit Alkyl oder Alkoxy mit je 1 bis 12 Kohlenstoffatomen, Phenyl, Hydroxyl, Halogen, Nitro, Cyano oder Carbalkoxy mit 2 bis 9 Kohlenstoffatomen, insbesondere $—CO_2CH_3$, Thiazol, Benzthiazol, Oxazol, Isoxazol oder Benzoxazol substituiert sein können.

Eine weitere Gruppe bevorzugter Verbindungen der Formel (1) sind solche, worin $R_2$ und $R_3$ ein Rest der Formel

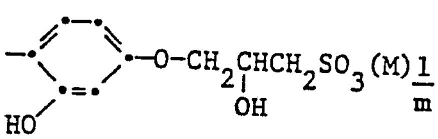 ,

worin M und die angegebene Bedeutungen haben, sind, $R_1$ dieselbe Bedeutung hat wie $R_2$ und $R_3$ oder $R_1$ Alkyl, mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 1 bis 9 Kohlenstoffatomen substituiert sein können, oder $—CH_2CH(OH)CH_2SO_3K$, Cyclopentyl, Cyclohexyl, Phenyl, Hydroxy, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder naphthyl, über ein Sauerstoff-, Schwefel- oder Stickstoffatom an den Triazinylrest gebundenes Alkyl mit 1 bis 12 Kohlenstoffatomen oder mit Methoxy, Hydroxy, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl, oder ein stickstoffhaltiger Heterocyclus mit 3 bis 6 Ringatomen ist.

Weitere besonders bevorzugte Verbindungen sind dadurch gekennzeichnet, dass $R_2$ und $R_3$ ein Rest der Formel

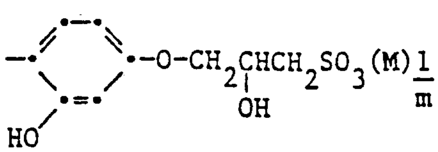

worin M Natrium, Kalium, Calcium, Magnesium, Ammonium oder Tetraalkylammonium mit je 1 bis 4 Kohlenstoffatomen in den Alkylresten und m 1 oder 2 ist, sind, und $R_1$ dieselbe Bedeutung hat wie $R_2$ und $R_3$ oder $R_1$ Alkyl mit 1 bis 12 Kohlenstoffatomen, Phenyl, über ein Sauerstoff- oder Schwefelatom oder Imino- oder Alkylimino mit 1 bis 4 Kohlenstofftomen im Akylrest an den Triazinylrest gebundenes Alkyl mit 1 bis 12 Kohlenstoffatomen oder Phenyl, oder ein stickstoffhaltiger heterocyclus mit 3 bis 6 Ringatomen ist, oder $R_2$ und $R_3$ die angegebene Bedeutung haben, und $R_1$ dieselbe Bedeutung hat wie $R_2$ und $R_3$ oder $R_1$ Alkyl, Alkoxy, Alkylamino oder Thioalkyl mit je 1 bis 12 Kohlenstoffatomen ist, wobei diese Reste mit Hydroxyl, Phenyl, Methoxy, Piperidinyl, Pyrrolidinyl, oder Carbalkoxy mit 2 bis 9 Kohlenstoffatomen substituiert sein können. Hierin sind jene Verbindungen von grossem Wert, worin $R_1$ Alkyl oder Alkoxy mit je 1 bis 4 Kohlenstoffatomen, Thioalkyl mit 8 bis 12 Kohlenstoffatomen oder Benzylamino ist oder auch $R_1$ ein Rest der Formel

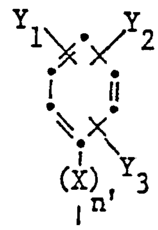

ist, worin n' 0 oder 1, X Sauerstoff, Schwefel, Imino oder Alkylimino mit 1 bis 4 Kohlenstoffatomen im Alkylrest ist, und $Y_1$, $Y_2$ und $Y_3$ unabhängig voneinander Wasserstoff, Alkyl oder Alkoxy mit je 1 bis 12 Kohlenstoffatomen, wobei die Alkyl- und Alkoxyreste mit Hydroxyl, Methoxy oder Carbalkoxy mit 2 bis 9 Kohlenstoffatomen substituiert sein können, $-CH_2CH(OH)CH_2SO_3K$, Cyclopentyl, Cyclohexyl, Phenyl, Hydroxyl, Sulfo, Halogen, Alkyl- oder Dialkylamino mit (je) 1 bis 4 Kohlenstoffatomen im Alkylrest, Nitro, Cyano oder Carbalkoxy mit 2 bis 4 Kohlenstoffatomen sind, oder X Sauerstoff oder Schwefel ist, $Y_1$, $Y_2$ und $Y_3$ unabhängig voneinander Wasserstoff, Alkyl oder Alkoxy mit je 1 bis 4 Kohlenstoffatomen, wobei die Alkyl- und Alkoxyreste mit Hydroxyl, Methoxy oder Carbalkoxy mit 2 bis 9 Kohlenstoffatomen substituiert sein können, Phenyl, Hydroxyl, Nitro, Cyano, $-CO_2CH_3$ oder Chlor sind, und n' die angegebene Bedeutung hat.

Ferner eignen sich jene Verbindungen, worin $R_1$ ein Heterocyclus der Formeln

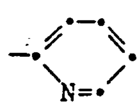 , 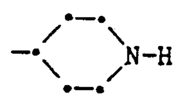 , 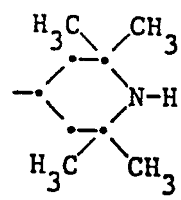 , 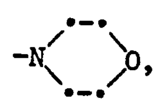 , 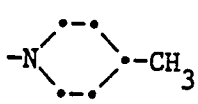 ,

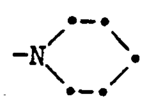 , 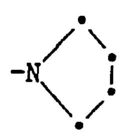 und 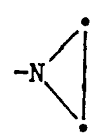 ,

vorzugswiese der Formeln

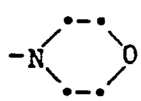 , 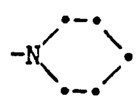 , 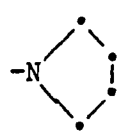 oder 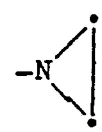 ist.

Insbesondere sind solche Verbindungen der Formel (1) geeignet, worin $R_2$ und $R_3$ ein Rest der Formel

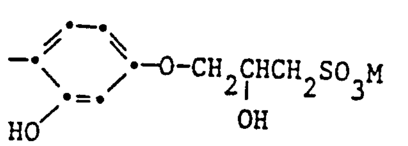 ,

worin M Natrium oder Kalium ist, sind, und $R_1$ Phenyl, Diphenyl, Tolyl oder p-Chlorphenyl ist.

Die Verbindungen der Formel (1) werden erfindungsgemäss nach folgendem Reaktionsschema hergestellt:

Stufe I:

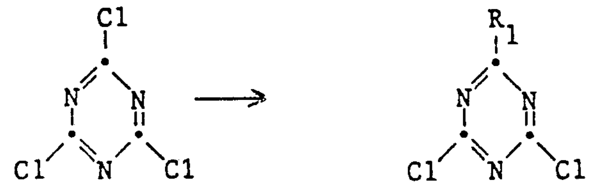

Stufe II:

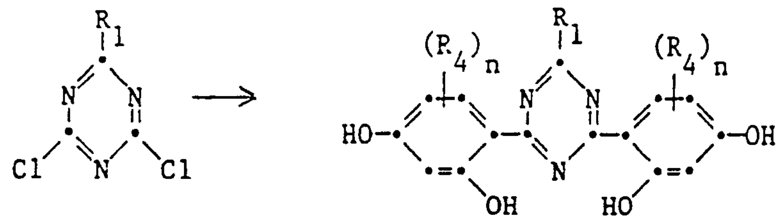

Stufe III:

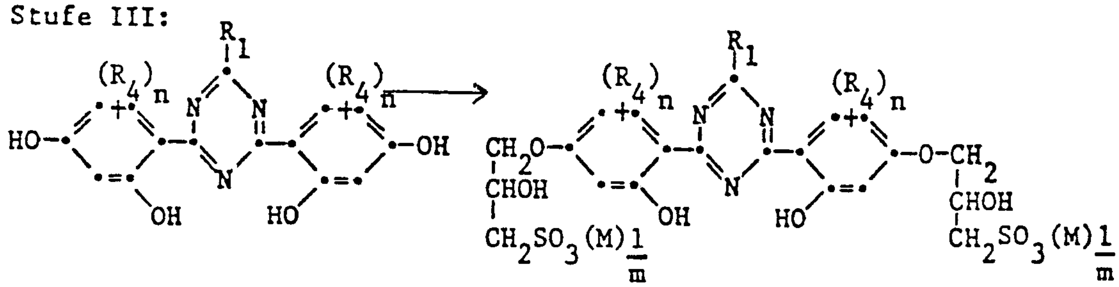

$R_1$, $R_4$, M, m und n haben die oben angegebenen Bedeutungen.

Die der Stufe I entsprechenden Umsetzungen sind an sich bekannt und z.B. in E. M. Smolin und L. Rapoport, The Chemistry of Heterocyclic Compounds: s- Triazines and Derivatives, Interscience Publishers Inc., New York, 1959, Seiten 1 bis 258 beschrieben.

Die chemischen Verfahrensschritte der Stufen II und III sind neu.

Die noch vorhandenen Chloratome am Triazinring werden in der Stufe II nach Friedel-Crafts in Gegenwart einer Lewis-Säure wie z.B. Aluminiumchlorid durch Resorcinylreste substituiert. Bisher wurden solche Reaktionen in Nitrobenzol als Lösungsmittel bei erhöhter Temperatur durchgeführt. Diese können sich aber insbesondere bei Reaktionstemperaturen von über 80°C als völlig unkontrollierbar erweisen.

Es wurde nun überraschenderweise gefunden, dass man durch Austausch von Nitrobenzol gegen Sulfolan (Tetramethylensulfon) als Lösungsmittel solche Reaktionen besser steuern kann. Die Verwendung von Sulfolan als Lösungsmittel bringt aber noch weitere Vorteile. Es wird nicht nur die Reaktionskontrolle verbessert, sondern es können auch die Zwischenprodukte in grösserer Reinheit und Ausbeute gewonnen werden. Ferner kann neben Aluminiumchlorid z.B. auch Titantetrachlorid als Lewis-Säure verwendet werden.

Erfindungsgemäss wird die Stufe II somit in der Weise ausgeführt, dass man das z.B. in 2-Stellung mit $R_1$ substituierte 4,6-Dichlortriazin mit vorzugsweise Aluminiumchlorid in einem Gemisch von Petroläther, vorzugsweise mit einem Siedebreich von 120 bis 140°C, und Sulfolan löst. Das Mischungsverhältnis Petroläther zu Sulfolan beträgt in der Regel 0:1 bis 10:1 (bezogen auf das Volumen). Dann wird die entsprechende Menge Resorcin in Sulfolan gelöst und der Reaktionsmischung langsam zugesetzt. Reaktionstemperatur und -dauer können in weiten Bereichen schwanken. Sie hängen weitgehend von dem bereits vorhandenen Substituenten $R_1$ab. Beispielsweise können Reaktionstemperaturen von 10 bis 95°C, vorzugsweise von 20 bis 80°C gewählt werden. Die Reaktionen sind in der Regel nach etwa 1 bis 6 Stunden beendet.

Die Zwischenprodukte der Stufe II können durch übliche Methoden, wie z.B. ausfällen, filtrieren, waschen und trocknen der kristallinen Reaktionsprodukte, isoliert werden.

In Stufe III erfolgt dann die basenkatalysierte Umsetzung der Zwischenprodukte au Stufe II mit der Verbindung der Formel

(2) 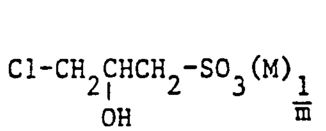 oder (3) 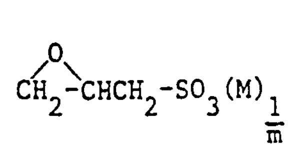 ,

worin M und m die angegebenen Bedeutungen haben, zu den Endprodukten der Formel (1). Als Katalysatoren können sowohl anorganische als auch organische Basen verwendet werden, wie (Erd-)-Alkalimetallhydroxide, -carbonate oder -methanolate, z.B. Natrium-, Kalium- oder Calciumhydroxid,

Natrium- oder Kaliumcarbonat oder -methanolat, sowie Pyridin oder Tetraalkylammoniumhydroxide wie Tetramethylammoniumhydroxid. Für die Umsetzung geeignete organische Lösungsmittel sind z.B. Alkohole wie Aethanol und Butanol, Ketone wie z.B. Aceton und Methyläthylketon, und Aether wie Diäthylenglykolmonomethyläther, 2-Methoxyäthanol und Dioxan. Die Reaktionsdauer beträgt im allgemeinen etwa 6 Stunden, wobei die Reaktionstemperatur in einem Bereich von 20 bis 150, vorzugweise zwischen 20 und 100°C liegt.

Durch die erfindungsgemässe Verwendung der Verbindungen der Formel (2) oder (3) in Stufe III umgeht man den Einsatz der gemäss US 3.444.164 verwendeten toxikologisch nicht unbedenklichen Sultone. Dadurch lässt sich die Herstellung der Verbindungen der Formel (1) ohne zusätzliche sicherheitstechnische Massnahmen durchführen. Die Verbindungen der Formel (1) fallen in grosser Reinheit und in guten Ausbeuten an.

Zur Herstellung solcher Verbindungen der Formel (1), worin $R_1$, $R_2$ und $R_3$ einen Rest der Formel

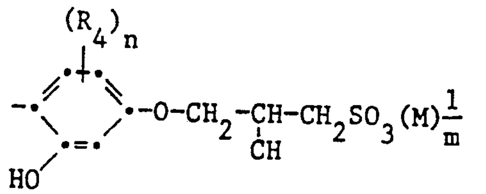

bedeuten, wird ausgehend von Trichlortriazin direkt gemäss Stufen (II) und (III) verfahren.

Auf die vorteilhafte Wirkung der erfindungsgemässen Verbindungen als UV-Absorber in photographischen Materialien wurde bereits eingangs hingewiesen. Die Verbindungen können sowohl in üblichen Chromogenmaterialien als auch in Silberfarbbleichmaterialien verwendet werden. Sie können in der Deckschicht, dem Träger, in Zwischenschichten, Bildfarbstoff enthaltenden Schichten und Silberhalogenidemulsionsschichten vorliegen. Vorzugsweise arbeitet man die Verbindungen in diejenige Schicht des betreffenden photographischen Materials ein, die direkt über der vor UV-Strahlen zu schützenden Schicht leigt. Geeignete Auftragsmengen für die erfindungsgemässen UV-Absorber liegen im Bereich von 100 bis 600, vorzugsweise von 200 bis 400 mg/m$^2$.

Im besonderen sei auf die gute Wasserlöslichkeit der erfindungsgemässen Verbindungen hingewiesen. Diese erlaubt eine frühzeitige Beurteilung der Silberfarbbleichmaterilien, in die sie eingearbeitet sind, schon während der Verarbeitung, d.h. im nassen Zustand. Bei Verwendung weniger gut wasserlöslicher Verbindungen, z.B. solchen gemäss US 3.444.164, ist eine entsprechende Beurteilung der Materialien nicht möglich, da diese im nassen Zustand eine starke Trübung aufweisen.

Prinzipiell lassen sich aber alle organischen Materialien, die durch UV-Strahlen geschädigt werden können, mit den erfindungsgemässen Verbindungen der Formel (1) schützen. Neben den schon erwähnten natürlichen und synthetischen Polymeren kommen also auch z.B. Farbstoffe bzw. Farbstoffzubereitungen, Kosmetika und Pharmazeutika in Frage.

Verfahrensvorschriften für Stufe II des erfindungsgemässen Verfahrens:

Beispiel 1

Herstellung der Verbindung der Formel

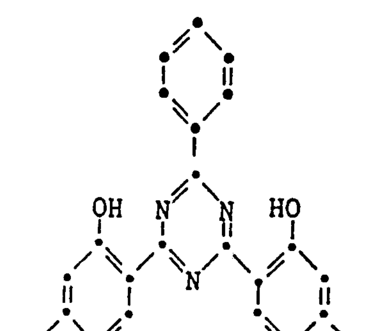

(101)

(Zwischenprodukt)

226 g 2-Phenyl-4,6-dichlor-s-triazin und 302 g Aluminiumchlorid werden in 620 ml Petroläther (Siedebereich 120 bis 140°C) suspendiert und mit 700 ml Sulfolan versetzt. Man erhitzt auf 40°C und gibt während 2,5 Studen 261 g Resorcin in 270 ml Sulfolan hinzu. Nach einer weiteren Stunde wird die untere Phase abgetrennt und in 4000 ml Methanol gegossen. Das Produkt wird durch Zugabe von 1350 ml Wasser ausgefällt, filtriert, nochmals in mit Salzsäure versetztem Wasser (pH 1,0) aufgeschlämmt und erneut

filtriert. Nach Trocknen bei 80°C erhält man 300 g (80% Ausbeute) der Ausgangsverbindung der Formel (101)

Die Elementaranalyse ergibt folgende Werte:

ber.: C 67,56%, N 11,26%, H 4,05%
gef.: C 66,28%, N 10,94%, H 4,16%

Schmelzpunkt: >240°C.

Beispiel 2

Herstellung der Verbindung der Formel

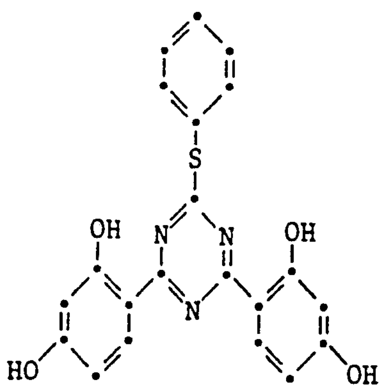

(102)

Ein Gemisch aus 5,2 g 2-Thiophenyl-4,6-dichloro-s-triazin und 5,6 g Aluminiumchlorid wird in 12 ml Petroläther (Siedebereich 120 bis 140°C) suspendiert. Dann gibt man 10 ml Sulfolan und eine Lösung von 4,8 g Resorcin in 6 ml Sulfolan langsam hinzu. Man lässt dann die Reaktionsmischung noch 2 Stunden bei 80°C nachreagieren. In 200 ml mit Salzsäure versetztem Wasser (pH 1,0) wird das Produkt ausgefällt. Nach zweimaligem Suspendieren des so erhaltenen Produktes in Wasser und Trocknen erhält man 4,8 g (60% Ausbeute) der Ausgangsverbindung der Formel (102).

Die Elementaranalyse ergibt folgende Werte:

ber.: C 59,39%, N 9,90%, H 4,07%
gef.: C 59,33%, N 9,97%, H 4,22%

Schmelzpunkt: > 240°C.

Beispiel 3

Herstellung der Verbindung der Formel

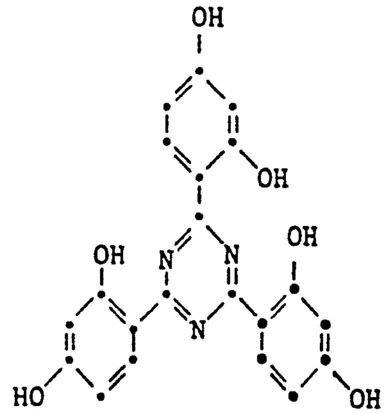

(103)

184,4 g Cyanurchlorid und 400 g Aluminiumchlorid werden in 700 ml Petroläther (Siedebereich 120 bis 140°C) suspendiert und mit 700 ml Sulfolan versetzt. Dann gibt man während 40 Minuten eine Lösung von 366 g Resorcin in 350 ml Sulfolan hinzu. Man rührt 5 Stunden bei 90 bis 95°C, trennt anschliessend die untere Phase ab und giesst diese in eine Mischung aus 4000 ml Methanol und 1000 ml Wasser. Die erhaltene Suspension wird noch 3 Stunden am Rückfluss erhitzt, dann filtriert und in 4000 ml salzsaurem Wasser (pH 1,0) suspendiert. Nach Filtration und Waschen mit heissem Wasser wird das Produkt in 3000 ml

Methanol suspendiert und heiss filtriert; man erhält 313 g (77% Ausbeute) der Ausgangsverbindung der Formel (103). 1 Mol dieser Verbindung enthält 1 Mol Kristallwasser.

Die Elementaranalyse ergibt folgende Werte:

ber.: C 59,52%; N 9,89%, H 4,25%
gef.: C 59,79%, N, 9,91%, H 4,33%

Schmelzpunkt: > 240°C.

Beispiel 4

Herstellung der Verbindung der Formel

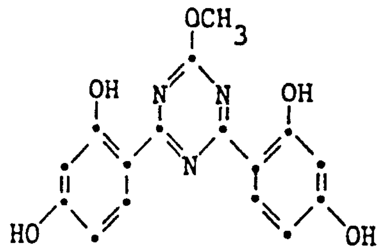

(104)

In einem Sulfierkolben werden 18 g 2-Methoxy-4,6-dichlor-s-triazin und 29,3 g Aluminiumchlorid in 60 ml Petroläther (Siedebereich 100 bis 120°C) vorgelegt und unter Rühren mit 50 ml Sulfolan bei 10°C versetzt. Nach 1 Stunde gibt man eine Lösung aus 24,2 Resorcin in 30 ml Sulfolan hinzu und rührt die Mischung noch 5 Stunden bei 20 bis 25°C. Danach wird das Reaktionsgemisch auf 0°C abgekühlt, mit 350 ml salzsaurem Wasser (pH 1,0) versetzt, 1 Stunde unter Rückfluss gekocht, filtriert und in 400 ml Methanol suspendiert. Nach Filtration und Trocknung erhält man 29,5 g (89,8% Ausbeute) der Ausgangsverbindung der Formel (104).

Die Elementaranalyse ergibt folgende Werte:

ber.: C 58,35%, N 12,75%, H 4,05%
gef.: C 58,03%, N 12,70%, H 3,99%

Schmelspunkt: >240°C.

Nach den in den Beispielen 1 bis 4 gezeigten Verfahren wurden die in der nachfolgenden Tabelle 1 auf eführten Verbindungen hergestellt.

Tabelle 1

(100) 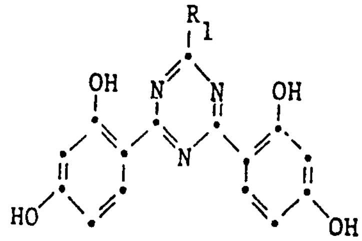 ° n $H_2O$

| Verbindung der Formel | $R_1$ | n | Ausbeute % / Reaktions-temp. °C | Schmelz-punkt °C | Elementaranalyse | | |
|---|---|---|---|---|---|---|---|
| | | | | | C | N | H |
| | | | | | %ber.<br>%gef. | | |
| (105) | 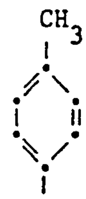 | 0,5 | 56/80 | > 250 | 67,53<br>67,48 | 10,78<br>10,77 | 4,50<br>4,64 |
| (106) | 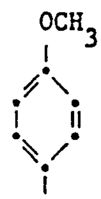 | 1,0 | 95/40 | > 250 | 62,90<br>61,00 | 10,00<br>9,78 | 4,52<br>4,64 |
| (107) | 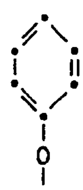 | 1,5 | 73/40 | > 210 | 60,31<br>60,10 | 10,04<br>10,10 | 4,39<br>4,50 |
| (108) | 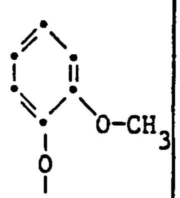 | 0,5 | 90/80 | > 250 | 61,05<br>60,01 | 9,70<br>9,93 | 4,50<br>4,50 |

Tabelle 1

(Fortsetzung)

(100) 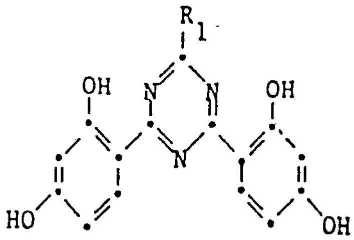 ° n $H_2O$

| Verbindung der Formel | $R_1$ | n | Ausbeute % / Reaktions-temp. °C | Schmelz-punkt °C | Elementaranalyse C | N | H |
|---|---|---|---|---|---|---|---|
| | | | | | %ber. %gef. | | |
| (109) | 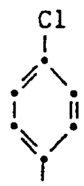 | 0,5 | 80/80 | > 250 | 61,15<br>57,73 | 10,18<br>9,49 | 3,54<br>3,56 |
| (110) | 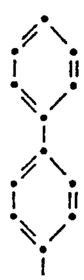 | 1,0 | 80/60 | > 250 | 69,06<br>70,87 | 8,95<br>9,52 | 4,56<br>4,60 |

Tabelle 1

(Fortsetzung)

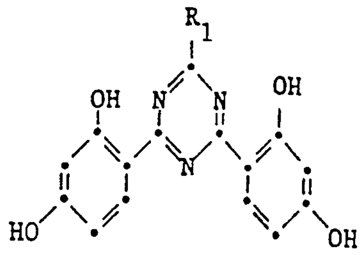

• $nH_2O$

| Verbindung der Formel | $R_1$ | n | Ausbeute % / Reaktions-temp. °C | Schmelz-punkt °C | Elementaranalyse C | H | N | Cl |
|---|---|---|---|---|---|---|---|---|
| | | | | | % ber. / % gef. | | | |
| (111) | 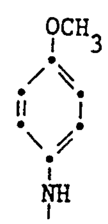 | 2 | 90/80 | >280 | 59.14<br>58.04 | 4.87<br>5.06 | 12.83<br>12.32 | -<br>- |
| (112) | 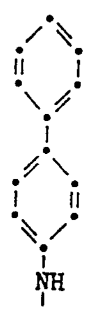 | 2 | 89/90 | >250 | 64.79<br>64.42 | 4.83<br>4.76 | 11.93<br>11.92 | -<br>- |
| (113) | 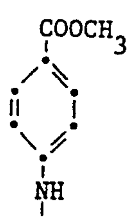 | 1 | 92/82 | >250 | 59.69<br>59.79 | 4.35<br>4.38 | 12.08<br>12.01 | -<br>- |
| (114) | 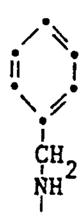 | 0 | 80/60 | >250 | 65.66<br>64.92 | 4.51<br>4.58 | 13.92<br>13.89 | -<br>- |

Tabelle 1

(Fortsetzung)

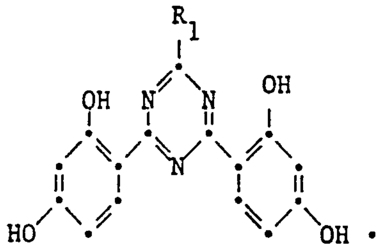

· $nH_2O$

| Verbindung der Formel | $R_1$ | n | Ausbeute % / Reaktionstemp. °C | Schmelzpunkt °C | Elementaranalyse C | H | N | Cl |
|---|---|---|---|---|---|---|---|---|
| | | | | | % ber. % gef. | | | |
| (115) | 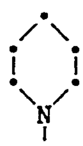 | 0 | 68/100 | >250 | 62.98<br>62.91 | 5.55<br>5.42 | 14.69<br>14.76 | -<br>- |
| (116) | 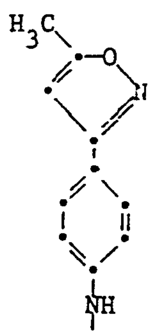 | 1 | 77/60 | >250 | 55.47<br>54.85 | 4.16<br>4.17 | 17.02<br>17.72 | -<br>- |
| (117) | 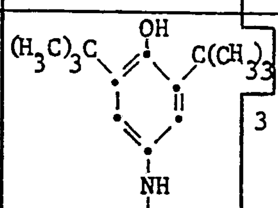 | 3 | 80/60 | >250 | 56.10<br>55.39 | 6.71<br>6.21 | 9.81<br>9.83 | -<br>- |
| (118) | 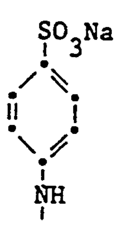 | 1 | 40/40 | >250 | 49.60<br>49.86 | 3.37<br>4.08 | 11.02<br>11.25 | -<br>- |

Tabelle 1

(Fortsetzung)

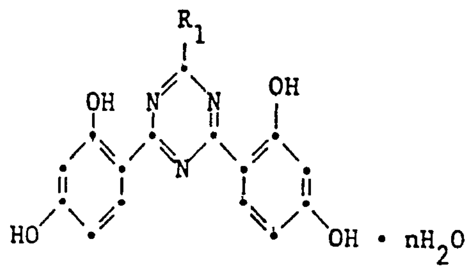

| Verbindung der Formel | $R_1$ | n | Ausbeute % / Reaktionstemp. °C | Schmelzpunkt °C | Elementaranalyse C | H | N | Cl |
|---|---|---|---|---|---|---|---|---|
| | | | | | % ber.<br>% gef. | | | |
| (119) | 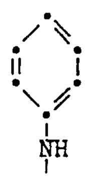 | 1 | 67/70 | >250 | 64.94<br>62.89 | 4.15<br>4.30 | 14.43<br>14.14 | -<br>- |
| (120) | 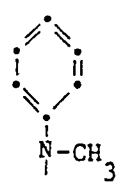 | 2 | 67/40 | > 250 | 60.26<br>60.51 | 5.05<br>5.15 | 12.78<br>12.93 | -<br>- |
| (121) | 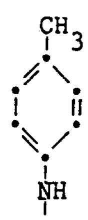 | 0,5 | 50/40 | > 250 | 64.22<br>63.99 | 4.65<br>4.68 | 13.61<br>14.12 | -<br>- |
| (122) | 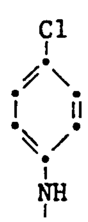 | 1 | 56/40 | > 250 | 57.21<br>56.71 | 4.34<br>3.80 | 12.70<br>13.22 | 8.04<br>9.81 |
| | | | | | | | | |

Tabelle 1

(Fortsetzung)

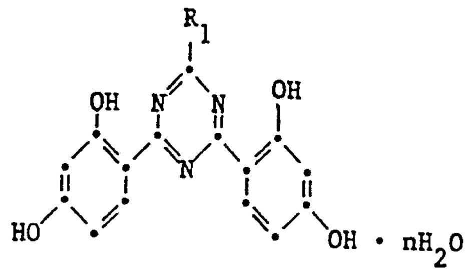

| Verbindung der Formel | $R_1$ | n | Ausbeute % / Reaktionstemp. °C | Schmelzpunkt °C | Elementaranalyse C | H | N | Cl |
|---|---|---|---|---|---|---|---|---|
| | | | | | % ber. / % gef. | | | |
| (123) | 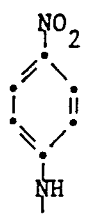 | 1 | 91/80 | > 250 | 55.87<br>55.37 | 3.79<br>3.70 | 15.50<br>16.09 | -<br>- |
| (124) | 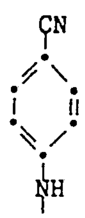 | 1 | 90/82 | > 250 | 61.25<br>60.04 | 3.97<br>4.32 | 16.27<br>15.53 | -<br>- |

Tabelle 2

(100a)

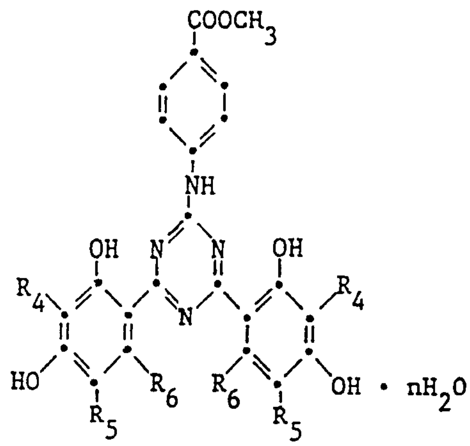

| Verbindung der Formel | $R_4$ | $R_5$ | $R_6$ | n | Ausbeute % Reaktionstemp. °C | Schmelzpunkt °C | Elementaranalyse C | H | N | Cl |
|---|---|---|---|---|---|---|---|---|---|---|
| | | | | | | | % ber. / % gef. | | | |
| (125) | H | H | OH | 1 | 88/80 | >250 | 55.76<br>54.60 | 4.07<br>4.39 | 11.29<br>10.57 | --<br>-- |
| (126) | H | H | $OCH_3$ | 3 | 89/90 | >250 | 53.57<br>53.58 | 5.035<br>4.65 | 9.99<br>10.01 | --<br>-- |
| (127) | $CH_3$ | H | H | 1 | 92/100 | >250 | 61.09<br>61.33 | 4.92<br>5.07 | 11.39<br>11.30 | --<br>-- |
| (128) | H | $COCH_3$ | H | 1 | 79/100 | >250 | 59.12<br>59.02 | 4.41<br>4.51 | 10.21<br>11.43 | --<br>-- |
| (129) | H | H | $CH_3$ | 1 | 56/80 | >250 | 65.86<br>66.97 | 5.04<br>4.91 | 10.01<br>10.37 | --<br>-- |

Tabelle 3

(100b)

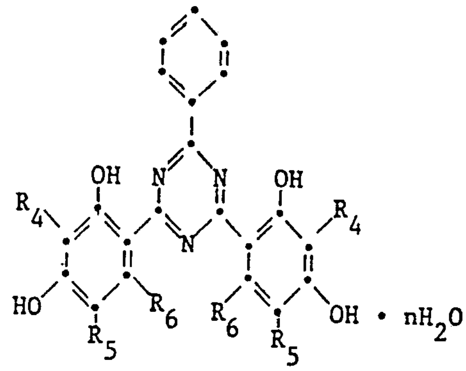

| Verbindung der Formel | $R_4$ | $R_5$ | $R_6$ | n | Ausbeute % / Reaktionstemp. °C | Schmelzpunkt °C | Elementaranalyse C | H | N | Cl |
|---|---|---|---|---|---|---|---|---|---|---|
| | | | | | | | % ber. / % gef. | | | |
| (130) | $CH_3$ | H | H | - | 84/100 | > 240 | 68.82<br>68.46 | 4.77<br>4.80 | 10.47<br>10.33 | --<br>-- |
| (131) | H | H | $OCH_3$ | 1 | 89/90 | > 240 | 61.19<br>61.09 | 4.68<br>4.64 | 9.3<br>9.4 | --<br>-- |
| (132) | H | H | OH | 1 | 91/90 | ~ 40 | 59.57<br>60.27 | 4.04<br>4.16 | 9.92<br>9.93 | --<br>-- |
| (133) | H | H | $CH_3$ | 1 | 56/80 | > 240 | 65.86<br>66.97 | 5.04<br>4.91 | 10.01<br>10.37 | --<br>-- |

Verfahrensvorschriften für Stufe III des erfindungsgemässen Verfahrens:

Beispiel 5

Herstellung der Verbindung der Formel

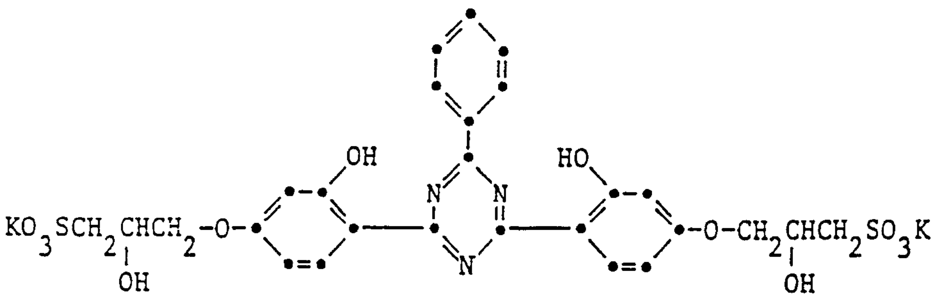

(201)

7,46 g 2-Phenyl-4,6-(2',4'-dihydroxy)-phenyl-s-triazin werden mit 3,36 g pulverisiertem Kaliumhydroxid in 110 ml Diäthylenglykolmonomethyläther bei Raumtemperatur gelöst. Nach Zugabe von 9,78 g Natrium-1-chlor-2-hydroxypropylsulfonat wird das Gemisch allmählich auf 100°C erhitzt. Nach 6 Stunden kühlt man die Reaktionsmischung auf 10°C, stellt mit Essigsäure einen pH-Wert von 7,0 ein und filtriert den Niederschlag ab. Nach Waschen mit Aethanol wird der Niederschlag in 80 ml Wasser aufgenommen und die Lösung auf 90°C erhitzt. Man gibt dann Aktivkohle hinzu, filtriert und kühlt das Filtrat auf 10°C. Mit 40 ml Aethanol wird das Produkt ausgefällt. Anschliessend wird filtriert und getrocknet. Man erhält 8,4 g (60% Ausbeute) der Verbindung der Formel (201).

Die Elementaranalyse ergibt folgende Werte:

| | | | | |
|---|---|---|---|---|
| ber.: | C 44,44%, | N 5,75%, | S 8,77%, | H 3,97% |
| gef.: | C 44,47%, | N 5,55%, | S 8,60%, | H 4,01% |

Schmelzpunkt: über 240°C.

Beispiel 6

Herstellung der Verbindung der Formel

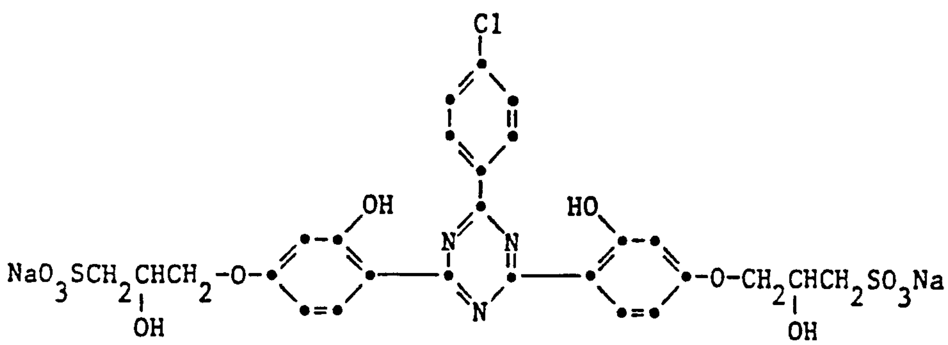

(202)

21,8 g 4''-Chlor-2,2',4',4'-tetrahydroxy-triphenyl-s-triazin werden in 200 ml 2-Methoxyäthanol suspendiert. Zu dieser Suspension werden 38,3 g 25 %ige methanolische Tetramethylammoniumhydroxydlösung zugetropft. Man erhitzt auf 50°C und hält die Reaktionsmischung bei dieser Temperatur, bis eine tiefrote Färbung eintritt. Nach Filtration werden 0,4 g Natriumjodid und 20,6 g 1-Chlor-2-hydroxy-propansulfonat (Natriumsalz) hinzugefügt. Man kocht die Reaktionsmischung 6 Stunden unter Rückfluss. Dann werden 4,1 g 1-Chlor-2-hydroxy-propansulfonat (Natriumsalz) und 0,08 g Natriumjodid hinzugegeben und weitere 22 Stunden bei Siedetemperatur gehalten. Nach Abkühlen der Reaktionsmischung wird das Produkt abfiltriert, mit Aethanol gewaschen, in einer Mischung aus 400 ml Aethanol und 5 ml Essigsäure wieder aufgeschlämmt, abgesaugt und bei 60°C im Vakuum getrocknet. Man erhält 35 g (87.5% Ausbeute) der Verbindung der Formel (202).

Die Elementaranalyse ergibt folgende Werde:

| | | | | |
|---|---|---|---|---|
| ber.: | C 40,53%, | N 5,25%, | S 8,01%, | H 4,03% |
| gef.: | C 40,58%, | N 5,21%, | S 7,79%, | H 4,08% |

Schmelzpunkt: über 240°C.

1 Mol Verbindung der Formel (202) enthält 4 Mol Kristallwasser.

Gemäss dem in Beispiel 5 gezeigten Verfahren wurden die in Tabelle 4 zusammengestellten Verbindungen hergestellt.

Tabelle 4

(200)

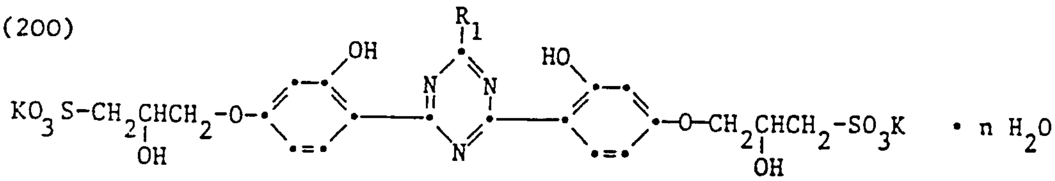

| Verbindung der Formel | $R_1$ | n | Absorptions-maximum(nm) | Elementaranalyse C | N | S | H |
|---|---|---|---|---|---|---|---|
| | | | | % ber. % gef. | | | |
| (203) | 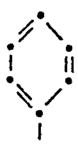 | 2 | 346 | 44,44<br>44,33 | 5,75<br>5,57 | 8,77<br>8,85 | 3,97<br>3,90 |
| (204) | 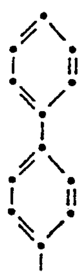 | 2 | 340 | 49,19<br>48,99 | 5,21<br>5,31 | 7,95<br>7,67 | 4,12<br>4,20 |
| (205) | 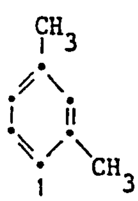 | 2 | 345 | 45,97<br>46,15 | 5,54<br>5,56 | 8,46<br>7,96 | 4,39<br>4,57 |

Tabelle 4

(Fortsetzung)

(200)

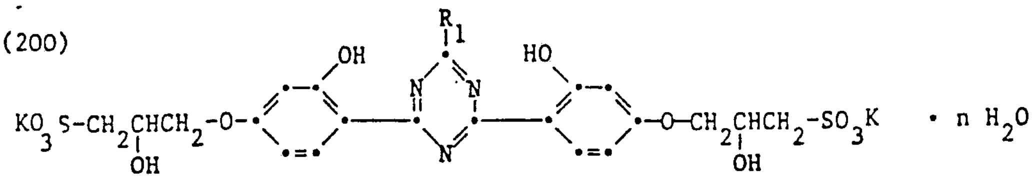

| Verbindung der Formel | $R_1$ | n | Absorptions-maximum(nm) | Elementaranalyse C | N | S | H |
|---|---|---|---|---|---|---|---|
| | | | | % ber. / % gef. | | | |
| (206) | 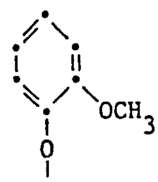 | 2 | 346 | 42,35<br>41,73 | 5,41<br>5,34 | 8,26<br>7,96 | 4,02<br>4,29 |
| (207) | 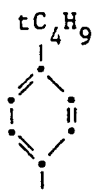 | 2 | 342 | 47,38<br>48,68 | 5,35<br>5,11 | 8,16<br>8,55 | 4,75<br>4,11 |
| (208) | $CH_3$ | 2 | 335 | 39,58<br>40,01 | 6,29<br>6,32 | 9,60<br>9,20 | 4,07<br>4,04 |
| (209) | 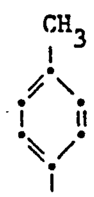 | 2 | 340 | 45,22<br>43,01 | 5,65<br>5,16 | -<br>- | 4,20<br>4,37 |
| (210) | 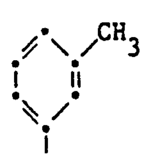 | 2 | 343 | 45,22<br>45,13 | 5,65<br>5,17 | 8,62<br>8,22 | 4,20<br>4,35 |
| (211) | $-SC_{12}H_{25}$ | 2 | 338 | 46,42<br>45,13 | 4,42<br>4,44 | 11,26<br>11,58 | 5,78<br>5,61 |

Nach den vorstehend beschriebenen Verfahren wurden auch die in Tabelle 5 gezeigten Verbindungen hergestellt.

Tabelle 5

(200a)

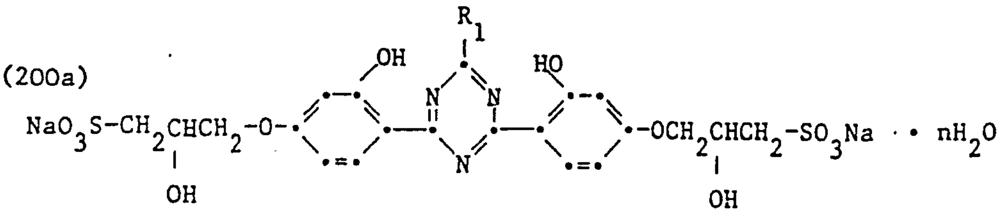

| Verbindung der Formel | $R_1$ | n | Absorptionsmaximum (nm) | Elementaranalyse C | H | N | S |
|---|---|---|---|---|---|---|---|
| | | | | % ber. / % gef. | | | |
| (212) | 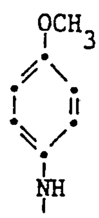 | 1 | 345 | 44.44<br>43.33 | 4.76<br>4.24 | 5.55<br>6.83 | 7.40<br>8.46 |
| (213) | 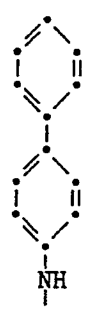 | 2 | 325 | 48.29<br>47.62 | 4.17<br>4.24 | 6.82<br>6.49 | 7.81<br>8.06 |
| (214) | 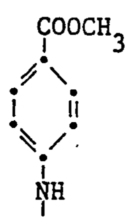 | 1 | 345 | 44.44<br>44.41 | 3.85<br>4.36 | 7.14<br>6.75 | 8.18<br>8.06 |
| (215) | 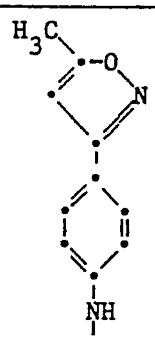 | 2 | 340 | 39.89<br>39.4 | 3.88<br>4.9 | 7.44<br>7.5 | 8.51<br>9.44 |

Tabelle 5

(Fortsetzung)

(200a)

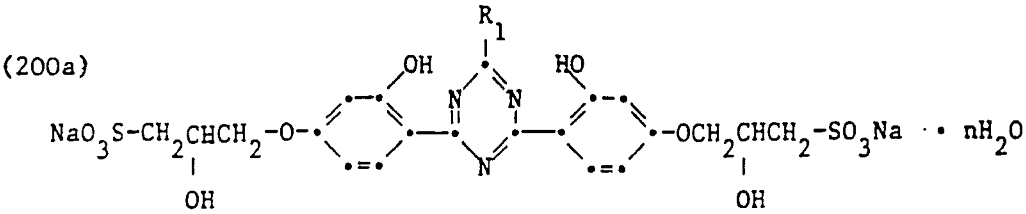

| Verbindung der Formel | $R_1$ | n | Absorptionsmaximum (nm) | Elementaranalyse | | | |
|---|---|---|---|---|---|---|---|
| | | | | C | H | N | S |
| | | | | % ber.<br>% gef. | | | |
| (216) | 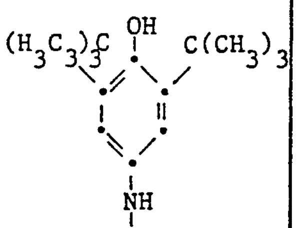 | 2 | 330 | 49.54<br>48.24 | 5.16<br>5.55 | 6.23<br>6.35 | 7.14<br>6.21 |
| (217) | 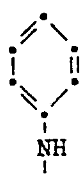 | 1 | 330 | 42.74<br>42.88 | 3.71<br>4.18 | 7.38<br>7.25 | 8.45<br>8.33 |
| (218) | 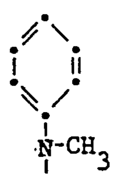 | - | 332 | 44.55<br>44.06 | 3.74<br>4.43 | 7.42<br>7.17 | 8.45<br>7.95 |
| (219) | 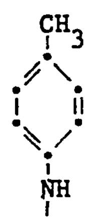 | 1 | 325 | 43.51<br>43.60 | 3.91<br>4.52 | 7.24<br>7.18 | 8.29<br>8.51 |

Tabelle 5

(Fortsetzung)

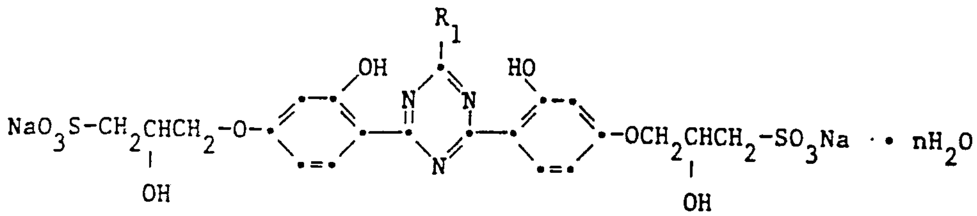

| Verbindung der Formel | $R_1$ | n | Absorptions-maximum (nm) | Elementaranalyse C | H | N | S |
|---|---|---|---|---|---|---|---|
| | | | | % ber.<br>% gef. | | | |
| (220) | 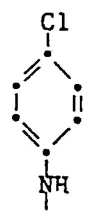 | 1 | 325 | 40.87<br>40.60 | 3.43<br>3.97 | 7.06<br>6.63 | 8.08<br>8.45 |
| (221) | 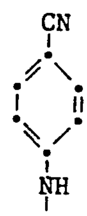 | 2 | 325 | 43.69<br>42.89 | 3.53<br>6.63 | 7.27<br>8.04 | 8.33<br>8.67 |
| (222) | 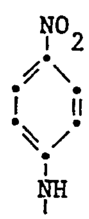 | 2 | 341 | 41.06<br>40.88 | 4.41<br>3.92 | 7.09<br>8.34 | 8.12<br>8.36 |
| (223) | 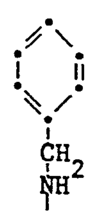 | - | 332 | 46.54<br>45.30 | 3.91<br>4.73 | 7.75<br>7.44 | 8.87<br>8.53 |
| (224) | 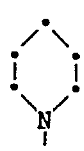 | - | 329 | 45.30<br>43.14 | 4.73<br>4.66 | 7.44<br>7.54 | 8.53<br>8.80 |

Das Absorptionsmaximum wurde aus dem UV-Spektrum einer die Verbindungen der Formel (203) bis (224) enthaltenden Gelatineschicht bestimmt. Diese Gelatineschicht war auf einen transparenten photographischen Träger gegossen worden.

Gemäss dem in Beispiel 5 gezeigten Verfahren wurden auch die in Tabelle 6 gezeigten Verbindungen hergestellt.

Tabelle 6

(300) 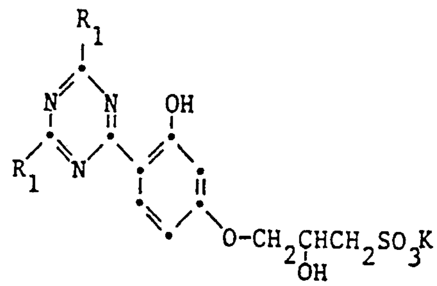

| Verbindung der Formel | $R_1$ | n | Absorptions-maximum(nm) | Elementaranalyse C | N | S | H |
|---|---|---|---|---|---|---|---|
| | | | | % ber.<br>% gef. | | | |
| (301) | 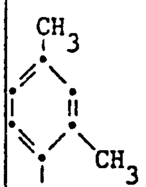 | 1 | 340 | 58,42<br>58,40 | 7,30<br>7,21 | 5,56<br>5,54 | 5,25<br>5,26 |
| (302) | 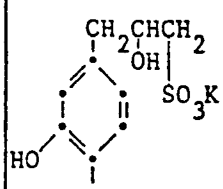 | 3 | 343 | 38,34<br>38,84 | 4,47<br>3,96 | 10,23<br>10,06 | 3,86<br>3,96 |

Das Absorptionsmaximum für diese Verbindungen wurde wie für die Verbindungen der Tabelle 4 gezeigt ermittelt.

Ferner wurden nach den vorstehend beschriebenen Verfahren die in den folgenden Tabellen 7 und 8 aufgeführten Verbindungen hergestellt.

Tabelle 7

(400)

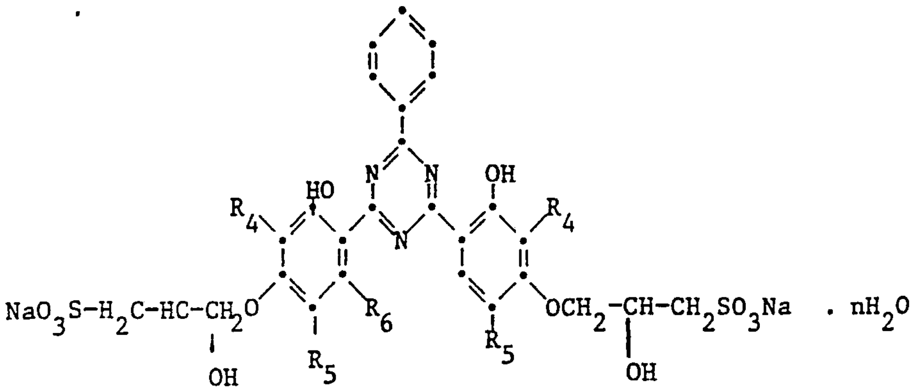

| Verbindung der Formel | $R_4$ | $R_5$ | $R_6$ | n | Absorptionsmaximum (nm) | Elementaranalyse C | H | N | S |
|---|---|---|---|---|---|---|---|---|---|
| | | | | | | % ber. / % gef. | | | |
| (401) | $CH_3$ | H | H | - | 345 | 48.26<br>47.78 | 4.05<br>4.61 | 5.82<br>5.53 | 8.88<br>8.30 |
| (402) | H | H | $OCH_3$ | - | 345 | 46.22<br>46.21 | 3.88<br>4.33 | 5.58<br>5.68 | 8.51<br>7.67 |
| (403) | H | H | OH | - | 345 | 40.65<br>40.46 | 3.66<br>4.17 | 5.51<br>4.71 | 8.42<br>9.62 |
| (404) | H | $COCH_3$ | H | - | 346 | 45.75<br>45.85 | 4.09<br>4.58 | 5.16<br>5.72 | 7.88<br>8.63 |
| (405) | H | H | $CH_3$ | - | 347 | 45.97<br>45.75 | 4.12<br>4.74 | 5.54<br>5.42 | 8.46<br>8.67 |

Tabelle 8

(500)

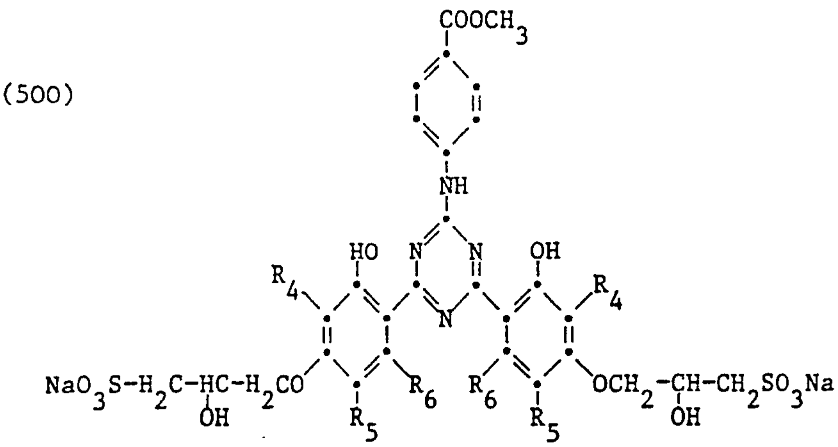

| Verbindung der Formel | $R_4$ | $R_5$ | $R_6$ | n | Absorptionsmaximum (nm) | Elementaranalyse C | H | N | S |
|---|---|---|---|---|---|---|---|---|---|
| | | | | | | % ber. / % gef. | | | |
| (501) | H | H | OH | 6 | 250 | 38.41<br>38.73 | 4.44<br>4.19 | 6.17<br>5.72 | 7.07<br>8.67 |
| (502) | H | H | $OCH_3$ | 2 | 345 | 43.16<br>43.31 | 4.20<br>4.58 | 6.49<br>6.33 | 7.43<br>7.60 |
| (503) | $CH_3$ | H | H | 2 | 345 | 44.82<br>44.84 | 4.36<br>4.82 | 6.74<br>6.33 | 7.71<br>7.99 |
| (504) | H | $\overset{O}{\overset{\parallel}{C}}-CH_3$ | H | 4 | 345 | 42.95<br>41.05 | 4.36<br>4.14 | 6.07<br>6.09 | 6.94<br>7.54 |

Beispiel 7

Herstellung der Verbindung der Formel

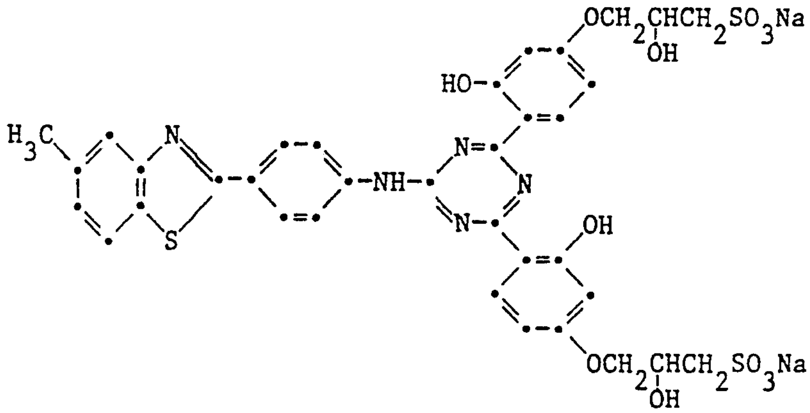

(505)

18,4 g Cyanurchlorid werden in 170 ml Aceton suspendiert und bei 0°C mit 2-(4-aminophenyl)-6-methylbenzothiazol versetzt. Die Reaktionsmischung wird noch 30 Minuten gerührt und anschliessend gibt man 5,3 g $Na_2CO_3$ zu. Dann werden 80 ml Eiswasser zugegeben und die Suspension wird kalt filtriert und bei 25°C im Vakuum getrocknet.

Das Rohprodukt wird in 250 ml Toluol suspendiert, 1 Stunde bei Rückfluss erwärmt, abfiltriert und anschliessend bei 40°C im Vakuum getrocknet. Man erhält 34 g (88%) der Ausgangsverbindung der Formel

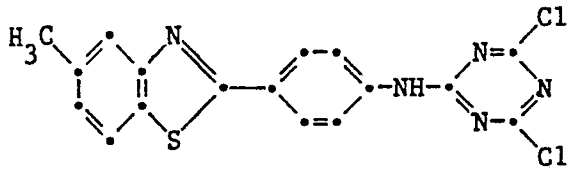

(505a)

Die Elementaranalyse für diese Verbindung ergab folgende Werte:

| | | | | | |
|---|---|---|---|---|---|
| ber.: | C 52,59 | H 2,86 | N 18,04 | S 8,26 | Cl 18,26 |
| gef. | C 52,16 | H 2,96 | N 17,71 | S 8,20 | Cl 18,39 |

Ein Gemisch aus 2-[2-(4-Aminophenyl)-6-methyl-benzothiazol]-4,6-dichloro-s-triazin (505a) und 5,6 g Aluminiumchlorid wird in 12 ml Petroläther (Siedebereich 120—140°C) suspendiert. Nach Zugabe von 16 ml Sulfolan und 4,8 g Resorcin wird die Reaktionsmischung noch 12 Stunden bei 60°C gerührt, nach dem Abkühlen auf Zimmertemperatur mit Wasser versetzt, filtriert und suspendiert in Wasser und dann in Methanol. Nach Filtration und Trocknung erhält man 8,7 g (85% Ausbeute) der Ausgangsverbindung der Formel

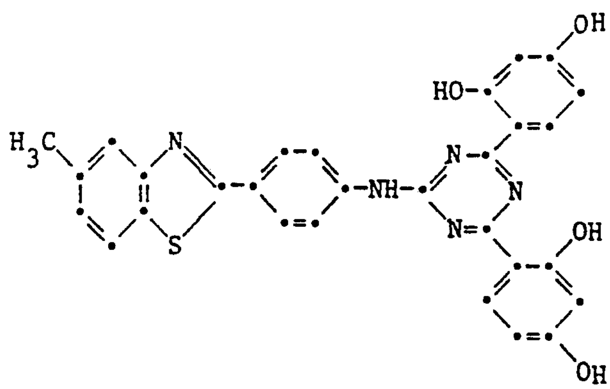

• $3H_2O$ (505b)

Die Elementaranalyse hierfür ergibt folgende Werte:

| | | | | |
|---|---|---|---|---|
| ber. | C = 59,07 | 4.69 | 11.87 | 5.43 |
| gef. | C = 59,35 | 4,13 | 13,84 | 6,40 |

10,7 g [2-(4-Aminophenyl-6-methyl-benzothiazol]-2,2',4,4'-tetrahydroxydiphenyl-s-triazin (505b) werden mit 2 g NaOH und 110 ml 2-Methoxyäthanol bei Zimmertemperatur gelöst. Nach Zugabe von 13,7 g Natrium-1-chlor-2-hydroxypropylsulfonat wird das Gemisch 3 Stunden bei 100°C gerührt, dann 1 Stunden bei 10°C gehalten und der Niederschlag abfiltriert. Nach dem Suspendieren in 210 ml Wasser und 105 ml Ethanol wird die Reaktionsmischung 1 Stunde bei Rückfluss gehalten, über Kieselgur filtriert und abgekühlt auf 0°C. Anschliessend wird das Produkt filtriert, in Ethanol suspendiert und isoliert. Man erhält nach dem Trocknen 5,9 g (35% Ausbeute) der Verbindung der Formel (505). Die Elementaranalyse ergibt folgende Werte:

| | | | | |
|---|---|---|---|---|
| ber. | C 47,14 | H 3,96 | N 7,85 | S 10,78 |
| gef. | C 47,20 | H 4,22 | N 8,28 | S 10,90 |

Beispiel 8

Vergleichsbeispiel

Es werden die erfindungsgemässen Verbindungen der Formel

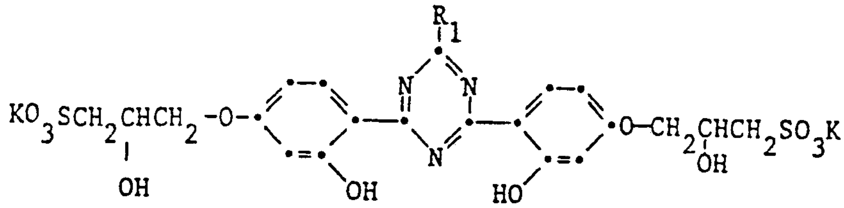

(200)

mit Verbindungen gemäss US 3 444 164 der Formel

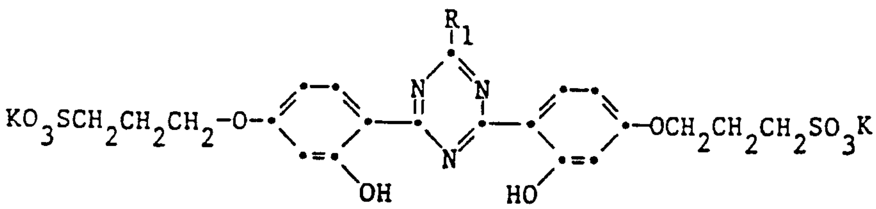

(600)

hinsichtlich ihrer Löslichkeit in Wasser und ihres Einflusses auf das Viskositätsverhalten wässriger Gelatinelösungen verglichen.

a) Bestimmung der maximalen Löslichkeit der genannten Verbindungen bei 20°C.

Tabelle 9

| $R_1$ | Löslichkeit (g /100 ml Wasser) der Verbindungen der Formel (200) | und (600) |
|---|---|---|
| 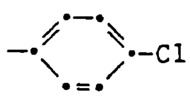 | 9 | 2 |
| 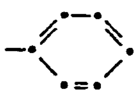 | 10 | 7 |
| 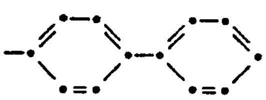 | 4 | 2 |
| 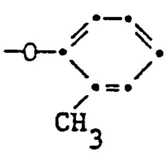 | 7 | 4 |
| $-SC_{12}H_{25}$ | 50 | 33 |

b) Einfluss vergleichbarer Mengen von Verbindungen der Formeln (200) und (600) auf das Viskositätsverhalten 5 %iger wässriger Gelatinelösungen vom pH-Wert 7.

Tabelle 10

| $R_1$ | g UV-Absorber/100 ml Gelatinelösung | Viskosität (Pa·sec) durch Verbindungen der Formeln (200) und (600) | |
|---|---|---|---|
| 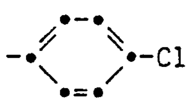 | 2 | 11,40 | 24,44 |
| 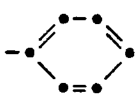 | 2 | 11,40 | 13,20 |
| 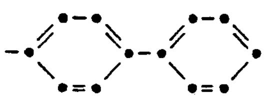 | 1 | 21,70 | 33,08 |
| $-SC_{12}H_{25}$ | 2 | 40,04 | 58,00 |

Ohne Zugabe von UV-Absorber beträgt die Viskosität der verwendeten Gelatinelösung 7,7 Pa·sec.

Aus den in den Tabellen 9 und 10 gezeigten Ergebnissen geht hervor, dass die erfindungsgemässen Verbindungen eine deutlich höhere Löslichkeit in Wasser besitzen und eine wesentlich geringere Viskositätserhöhung in wässrigen Gelatinelösungen verursachen als die Verbindungen gemäss US 3 444 164. Durch diese vorteilhaften Eigenschaften bieten sich die erfindungsgemässen Verbindungen insbesondere zur Verwendung in Gelatineschichten photographischer Materialien an.

Anwendungsbeispiele:

Beispiel 9

Auf einen transparenten photographischen Träger wird eine Silberhalogenidemulsion vergossen, die soviel Magentakuppler der Formel

Formel

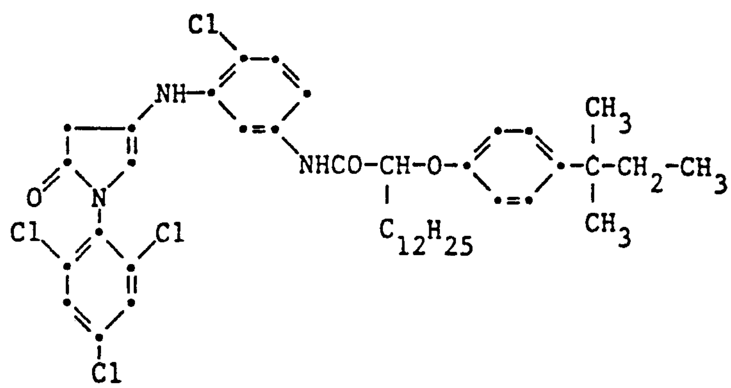

(701)

enthält, dass nach Belichtung und üblicher chromogener Verarbeitung des Materials eine Magentadichte von 1,0 erhalten wird. Auf dieses Material wird eine Gelatineschicht gegossen (Auftragsgewicht 5 g/m²), die 500 mg/m² des UV-Absorbers der Formel (204) enthält. Das gleiche Material wird hergestellt, jedoch ohne UV-Absorber in der Gelatineschicht.

Beide Materialien belichtet man in einer alternierend trockenen und feuchten Atmosphäre, wobei die Materialien einer Lichtenergie von 20 kJ/cm² ausgesetzt werden.

Die nach der Belichtung gemessenen Restdichten an Magentafarbstoff betragen 0,82 für das Material mit dem erfindungsgemässen UV-Absorber, aber nur 0,20 für das Material ohne UV-Absorber.

Beispiel 10

Auf einen transparenten photographischen Träger wird eine Gelatineschicht gegossen, die soviel Magentabildfarbstoff der Formel

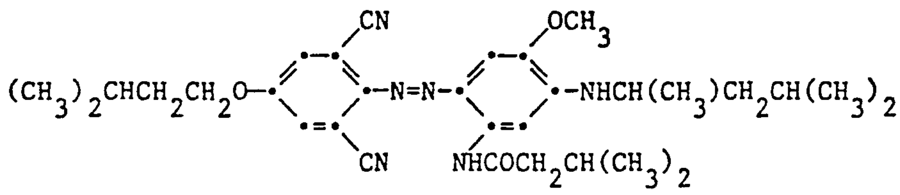

(702)

enthält, dass sich eine Magentadichte von 1,0 ergibt. Auf diese Schicht wird eine Gelatineschicht gegossen (Auftragsgewicht 5 g/m²), die 500 mg/m² des UV-Absorbers der Formel (204) enthält.

Das gleiche Material wird hergestellt, jedoch ohne UV-Absorber in der Gelatineschicht.

Beide Materialien belichtet man in einer alternierend trockenen und feuchten Atmosphäre, wobei die Materialien einer Lichtenergie von 80 kJ/cm² ausgesetzt werden.

Die nach der Belichtung gemessenen Restdichten an Magentafarbstoff betragen 0,86 für das Material mit dem erfindungsgemässen UV-Absorber, aber nur 0,60 für das Material ohne UV-Absorber.

Beispiel 11

Es wird ein photographisches Material (A) für das Silberfarbbleichverfahren mit folgendem Aufbau herstellt:

Gelatine-Schutzschicht

Blauempfindliche, jodidfreie AgBr-Emulsion

Gelbfarbstoff (705), blauempfindliche, jodidfreie AgBr-Emulsion

Gelbfilter: Gelbes Ag-Hydrosol (40 mg/m²)

Grünempfindliche AgBr/AgJ-Emulsion

Purpurfarbstoff (704), grünempfindliche AgBr/AgJ-Emulsion

Zwischenschicht (Gelatine)

Blaugrünfarbstoff (703), rotempfindliche AgBr/AgJ-Emulsion

Rotempfindliche AgBr/AgJ-Emulsion

Cellulosetriacetat-Träger, weissopak

Rückschicht, Gelatine

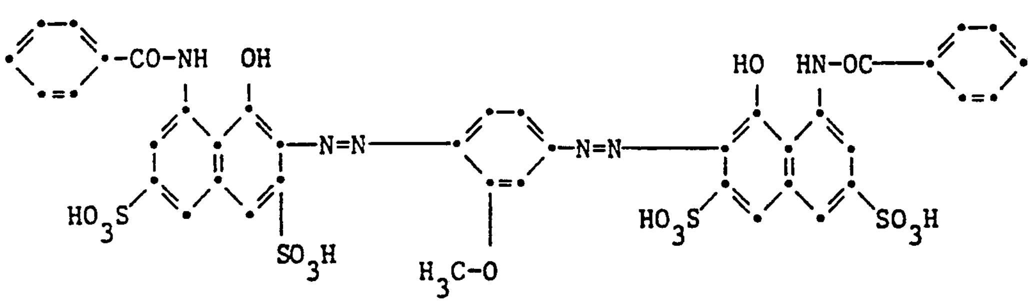

(703)

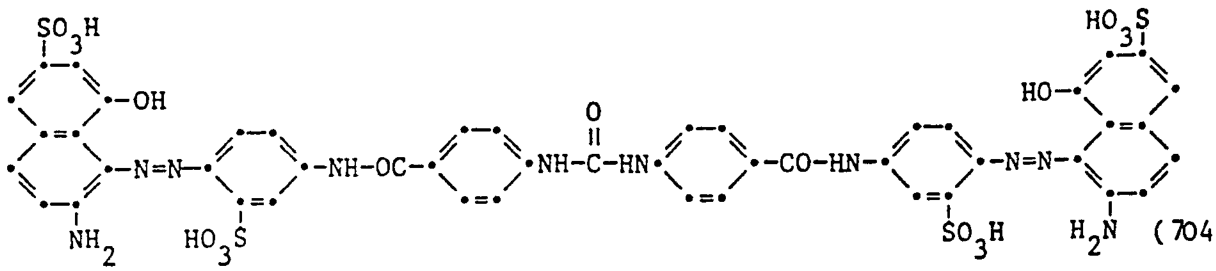

(704)

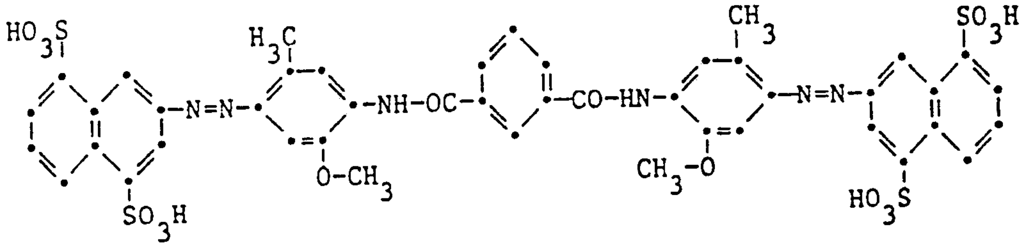

(705)

Die jodidhaltigen Emulsionsschicht enthalten Silberhalogenidkristalle mit 2,6 Mol-% Silberjodid und 97,4 Mol-% Silberbromid. Die Bildfarbstoffe werden in einer solchen Konzentration verwendet, dass ihre Remissionsdichte je 2,0 beträgt: der Gesamt-Silbergehalt der 22 µm dicken Schichten beträgt 2,0 g/m².

Ein weiteres photographisches Material (B) wird hergestellt. Material B ist gleich aufgebaut wie Material A, es enthält jedoch 210 mg/m² des UV-Absorbers der Formel (201) in der Gelatine-Schutzschicht.

Diese Materialien werden belichtet und wie folgt verarbeitet:

1. Silberentwicklung während 3 Minuten bei 30°C in einem Bad der folgenden Zusammensetzung:

| | |
|---|---|
| Aethylendiamintetraessigsäure, (Natriumsalz) | 4 g |
| Kaliumsulfit | 19,9 g |
| Natriumsulfit, wasserfrei | 38,0 g |
| Hydrochinon | 8,0 g |
| 1-Phenyl-4-methylpyrazolidon | 0,5 g |
| Kaliumkarbonat, wasserfrei | 19,5 g |
| Kaliumbikarbonat | 13.3 g |
| Kaliumbromid | 3,5 g |
| Benztriazol | 1,0 g |
| Aethylcellosolve | 57,4 g |
| Natriumthiosulfat, wasserfrei | 0,9 g |
| Wasser bis | 1000 ml |

2. Wässern während 1 Minute.

3. Gleichzeitige Farbstoff- und Silberbleichung während 3 Minuten bei 30°C in einem Bleichbad der folgenden Zusammensetzung:

| | |
|---|---|
| Schwefelsäure (100%) | 41,8 g |
| m-Nitrobenzolsulfonsäure, (Natriumsalz) | 7,5 g |
| Aethylcellosolve | 57,4 g |
| 2,3,6-Trimethylchinoxalin | 1,1 g |

| | |
|---|---|
| Kaliumjodid | 9,0 g |
| Bis-(β-Cyanoäthyl)-sulfoäthylphosphin, (Natriumsalz) | 2,9 g |
| Wasser bis | 1000 ml |

4. Wässern während 1 Minute.
5. Fixieren während 3 Minuten bei 30°C in einem Fixierbad der Zusammensetzung:

| | |
|---|---|
| Ethylendiamin-tetraessigsäure (Natriumsalz) | 3 g |
| Ammoniumthiosulfat 60% | 333 g |
| Ammoniumbisulfit 60% | 75 g |
| Ammoniak | 21 ml |
| Wasser auf | 1000 ml |

6. Wässern während 4 Minuten und Trocknen der Materialien.

Bestimmt man die photographische Empfindlichkeit der Materialien A und B im Bereich zwischen 300 und 450 nm, so erhält man die in Tabelle II angegebenen Werte.

Tabelle 11

Relative photographische Empfindlichkeit als Funktion der (in nm gemessenen) Wellenlänge

| Material | UV-Gebiet | | | | Sichtbares Spektralgebiet | |
|---|---|---|---|---|---|---|
| | 314 | 336 | 355 | 378 | 400 | 425 |
| A | 5.10 | 4.78 | 4.93 | 4.85 | 4.69 | 4.43 |
| B | 4.89 | 4.35 | 4.44 | 4.63 | 4.66 | 4.42 |
| A - B | 0.21 | 0.43 | 0.49 | 0.22 | 0.03 | 0.01 |

Es ist klar ersichtlich, dass Material B im UV-Bereich deutlich weniger empfindlich ist als Material A. Im sichtbaren Bereich (400, 425 nm) hat sich die Empfindlichkeit dagegen nicht geändert.

Die Bestimmung der photographischen Empfindlichkeit ist in T. H. James, The Theory of The Photographic Process, 4th ed., 1977, Seite 510, beschrieben.

Beispiel 12

Unbelichtete Filmstreifen der in Beispiel 11 beschriebenen Materialien A und B werden mit dem Licht einer Gleichspannungs-Coronaentladung belichtet, wobei die Belichtungszeiten zwischen 0,1 und 100 Sekunden variiert werden. Die so belichteten Filmstreifen werden, wie in Beispiel 11 beschrieben, verarbeitet. Man misst die Abnahme der Maximaldichte im blauen Spektralbereich und ermittelt die Expositionszeit, die nötig ist, um eine Dichteabnahme von 15% zu verursachen. Während Material A schon nach 0,3 Sekunden Exposition 15% der Maximaldichte der blauempfindlichen Schicht verloren hat, kann Material B dreimal so lange, also 0,9 Sekunden lang belichtet werden bis der gleiche Maximaldichteverlust eintritt.

Beispiel 13

In gewissen Fällen wird eine ausreichend grosse Wasserlöslichkeit der in photographischen Materialien verwendeten UV-Absorber verlangt. Dieses Beispiel zeigt, wie man durch Wahl eines geeigneten Substituenten $R_1$ beispielsweise in den erfindungsgemässen Verbindungen der Formel (200) dieser Anforderung gerecht wird. Je nach Art des Substituenten $R_1$ können die UV-Absorber nämlich wasserlöslich oder -unlöslich gemacht werden. So kann man erreichen, dass der UV-Absorber während der Verarbeitung des Materials entweder im Material verbleibt oder ausgewaschen wird.

Auf einen weissen, polyäthylenbeschichteten Papierträger werden Gelatineschichten aufgetragen, die pro $m^2$ Schichtträgerfläche 7,2 g Gelatine, 0,06 g des Härtungsmittels 2-(N-Methylmorpholino)-4-amino-6-hydroxy-triazintetrafluoroborat und 0,25 g eines UV-Absorbers der Formel (200) enthält, worin $R_1$ die in Tabelle 12 angegebenen Bedeutungen hat.

Tabelle 12

| Gelatineschicht Nr. | $R_1$ |
|---|---|
| 1 | ohne UV-Absorber (Vergleich) |
| 2 | 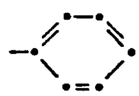 |
| 3 | 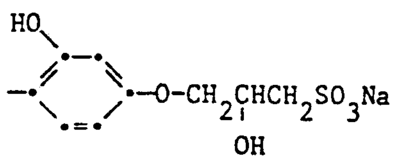 |
| 4 | 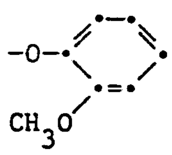 |

An diesen Gelatineschichten wird mit einem geeigneten Farbmessgerät (CHROMA-METER II von MINOLTA) der Farbort bestimmt. Die Beleuchtung erfolgt dabei mit tageschlichtähnlichem, UV-haltigem Weisslicht (Normlichtart D 65). Aus den Farbkoordinaten Y, x, y lässt sich der Weissgrad W nach Color Research and Application 6, (1981), 107 wie folgt berechnen:

$$W = Y + 800\,(0.3138 - x) + 1700\,(0.3310 - y).$$

Ist die Lichtabsorption des UV-Absorbers bei Wellenlängen oberhalb 400 nm nicht vernachlässigbar klein, so sinkt der Weissgrad unter denjenigen des unbegossenen Trägers und die Schicht erhält einen Gelbstich, der um so stärker ausfällt, je ausgeprägter die in das Spektralgebiet des sichtbaren Lichts hereinreichende Absorption des UV-Absorbers ist.

Im Gegensatz zu dem genannten polyäthylenbeschichteten Papierträger enthalten weiss-opake Polyesterträger einen optischen Aufheller, der den unsichtbaren UV-Anteil des einfallenden Lichts in sichtbares Licht des blauen Spektralgebiets verwandelt und dadurch den Weisseindruck des Trägers verbessert. Dieser Aufhelleffekt wird durch UV-Absorber unvermeidlicherweise weitgehend unterdrückt. Dadurch verschlechtert sich in diesem Fall der Weissgrad auch dann, wenn die Absorption der UV-Absorber im sichtbaren Spektralgebiet vernachlässigt werden kann.

Das Ausmass der beiden eben diskutierten Effekte ist, wie die in der folgenden Tabelle 13, Spalte 4, zusammengestellten Messwerte zeigen, recht erheblich, wenn die Prüfschichten vor ihrer Ausmessung keine weitere chemische Behandlung erleiden. Werden sie dagegen, wie in Beispiel 11 beschrieben, einem Verarbeitungsprozess für das Silberfarbbleichverfahren unterworfen, so diffundiert der UV-Absorber

wegen seiner guten Wasserlöslichkeit teilweise in die Verarbeitungsbäder. Die dadurch bedingte Aenderung des Gehalts an UV-Absorber in den Prüfschichten lässt sich spektralphotometrisch bestimmen; sie liegt bei den in Tabelle 13, Spalte 4, angegebenen Beispielen zwischen 94 und 97% des Anfangsgehalts. Der Weissgrad dieser Schichten unterscheidet sich also erwartungsgemäss nur noch wenig von demjenigen einer UV-Absorberfreien Schicht (vgl. Spalte 3 der Tabelle 13).

Tabelle 13

Weissgrad (W) weissopaker Träger mit den Schichten von Tab. 12 und nachfolgender Verarbeitung nach dem Silberfarbbleichverfahren.
ΔG = Prozentuale Abnahme des UV-Absorber-Auftrages bei der Verarbeitung.

| 1 | 2 | 3 | | 4 |
|---|---|---|---|---|
| Träger | Gelatine-Schicht Nr. | W vor | nach Verarbeitung | ΔG % |
| Polyäthylenbeschichtetes Papier (nicht aufgehellt) | 1 | 101 | 101 | - |
| | 2 | 94 | 99 | 94 |
| | 3 | 89,1 | 101 | 97 |
| | 4 | 97,2 | 99,8 | 96 |
| Polyester weiss-opak (optisch aufgehellt | 1 | 110 | 110 | - |
| | 2 | 103 | 109 | 94 |
| | 3 | 98,1 | 109,5 | 97 |
| | 4 | 105,3 | 109,6 | 96 |

## Patentansprüche

1. Verbindungen der Formel

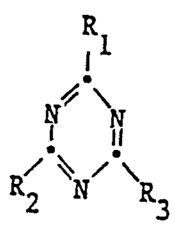

(1)

worin mindestens einer der Substituenten $R_1$, $R_2$ und $R_3$ ein Rest der Formel

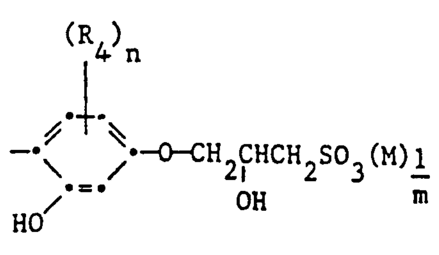

ist, worin M ein Alkali- oder Erdalkalimetall, Ammonium oder Tetraalkylammonium, worin die Alkylreste unabhängig voneinander 1 bis 4 Kohlenstoffatome enthalten, m 1 oder 2, $R_4$ Alkyl oder Alkoxy mit je 1 bis 4 Kohlenstoffatomen, Hydroxyl oder einen Rest der Formel —COR, worin R Alkyl mit 1 bis 8 Kohlenstoffatomen oder Phenyl bedeutet, und n 0, 1, 2 oder 3 ist, und der übrige Substituent bzw. die übrigen Substituenten unabhängig voneinander Alkyl, mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl ist bzw. sind, wobei dieser Substituent bzw. diese Substituenten auch über ein Sauerstoff-, Schwefel- oder Stickstoffatom an den Triazinylrest gebunden sein kann bzw. sein können, wobei ein über ein Stickstoffatom an den Triazinylring gebundener Phenylrest zusätzlich noch mit Thiazol-, Benzthiazol-, Oxazol-, Isoxazol-, und Benzoxazolgruppen substituiert sein kann, oder dieser Substituent bzw. diese Substituenten ein stickstoffhaltiger Heterocyclus mit 3 bis 6 Ringatomen ist bzw. sind.

2. Verbindungen nach Anspruch 1, dadurch gekennzeichnet, dass $R_2$ und $R_3$ ein Rest der Formel

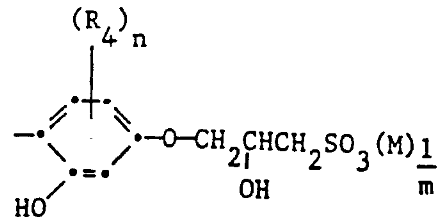

sind, worin $R_4$ Methyl, Methoxy, Hydroxyl oder Acetyl, n 1, 2 oder 3 ist, M und m die in Anspruch 1 angegebene Bedeutung haben und $R_1$ Alkyl mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)-Alkylamino mit (je) 1 bis 4 Kohlenstoffatomen in Alkylrest, Nitro, Cyano, Carbalkoxy mit 2 bis 9 Kohlenstoffatomen oder Sulfogruppen substituiertes Phenyl oder Naphthyl, über ein Sauerstoff-, Schwefel- oder Stickstoffatom an den Triazinylrest gebundenes Alkyl mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl, oder ein stickstoffhaltiger Heterocyclus mit 3 bis 6 Ringatomen ist.

3. Verbindungen nach Anspruch 2, dadurch gekennzeichnet, dass $R_1$ Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl oder über ein Sauerstoff-, Schwefel- oder Stickstoffatom an den Triazinylrest gebundenes Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl ist.

4. Verbindungen nach Anspruch 3, dadurch gekennzeichnet, dass $R_1$ Phenyl, Anilino oder N-Alkyl-Anilino ist, wobei diese Reste mit Alkyl oder Alkoxy mit je 1 bis 12 Kohlenstoffatomen, Phenyl, Hydroxyl, Halogen, Nitro, Cyano oder Carbalkoxy mit 2 bis 9 Kohlenstoffatomen, Thiazol-, Benzthiazol-, Oxazol-, Isoxazol- oder Benzoxazolgruppen substituiert sein können.

5. Verbindungen nach Anspruch 4, dadurch gekennzeichnet, dass $R_1$ Phenyl oder ein Rest der Formel

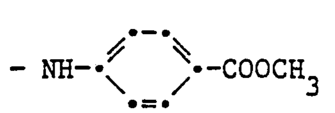

ist.

6. Verbindungen nach Anspruch 1, dadurch gekennzeichnet, dass $R_2$ und $R_3$ Reste der Formel

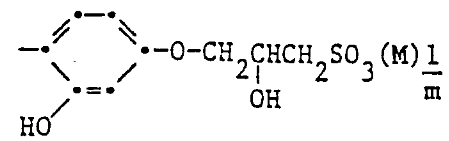

sind, M und m die in Anspruch 1 angegebene Bedeutung haben, $R_1$ dieselbe Bedeutung hat wie $R_2$ und $R_3$ oder $R_1$ Alkyl, mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl, über ein Sauerstoff-, Schwefel- oder Stickstoffatomen an den Triazinylrest gebundenes Alkyl mit 1 bis 12 Kohlenstoffatomen oder mit Methoxy, Hydroxyl, Phenyl, Piperidinyl, Pyrrolidinyl oder Carbalkoxy mit 2 bis 9 Kohlenstoffatomen substituiertes Alkyl mit 1 bis 12 Kohlenstoffatomen, Phenyl oder Naphthyl oder mit Alkyl oder Alkoxy mit je 1 bis 12 Kohlenstoffatomen, die mit Hydroxyl, Methoxy oder Carbalkoxy mit 2 bis 9 Kohlenstoffatomen substituiert sein können, oder $—CH_2CH(OH)CH_2SO_3K$, Cyclopentyl, Cyclohexyl, Phenyl, Hydroxyl, Halogen, (Di-)Alkylamino mit (je) 1 bis 4 Kohlenstoffatomen im Alkylrest, Nitro, Cyano, Carbalkoxy mit 2 bis 9 Kohlenstoffatomen oder Sulfogruppen substituiertes Phenyl oder Naphthyl, oder ein stickstoffhaltiger Heterocyclus mit 3 bis 6 Ringatomen ist.

7. Verbindungen nach Anspruch 6, dadurch gekennzeichnet, dass $R_2$ und $R_3$ ein Rest der Formel

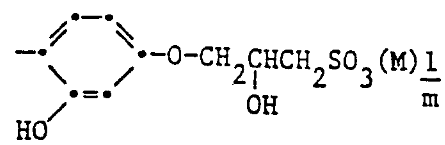 ,

sind, M Natrium, Kalium, Calcium, Magnesium, Ammonium oder Tetraalkylammonium mit je 1 bis 4 Kohlenstoffatomen in den Alkylresten und m 1 oder 2 ist, und $R_1$ dieselbe Bedeutung hat wie $R_2$ und $R_3$ oder $R_1$ Alkyl mit 1 bis 12 Kohlenstoffatomen, Phenyl, durch Sauerstoff, Schwefel oder Imino- oder Alkylimino mit 1 bis 4 Kohlenstoffatomen im Alkylrest an den Triazinylrest gebundenes Alkyl mit 1 bis 12 Kohlenstoffatomen oder Phenyl, oder ein stickstoffhaltiger Heterocyclus mit 3 bis 6 Ringatomen ist.

8. Verbindungen nach Anspruch 7, dadurch gekennzeichnet, dass $R_2$ und $R_3$ die in Anspruch 7 angegebene Bedeutung haben, und $R_1$ dieselbe Bedeutung hat wie $R_2$ und $R_3$ oder $R_1$ Alkyl, Alkoxy, Alkylamino oder Thioalkyl mit je 1 bis 12 Kohlenstoffatomen ist, wobei diese Reste mit Hydroxyl, Phenyl, Methoxy, Piperidinyl, Pyrrolidinyl, oder Carbalkoxy mit 2 bis 9 Kohlenstoffatomen substituiert sein können.

9. Verbindungen nach Anspruch 8, dadurch gekennzeichnet, dass $R_1$ Alkyl oder Alkoxy mit je 1 bis 4 Kohlenstoffatomen, Benzylamino oder Thio alkyl mit 8 bis 12 Kohlenstoffatomen ist.

10. Verbindungen nach Anspruch 7, dadurch gekennzeichnet, dass $R_1$ ein Rest der Formel

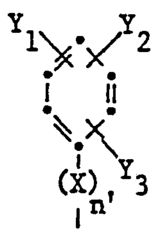

ist, worin n' 0 oder 1, X Sauerstoff, Schwefel, Imino oder Alkylimino mit 1 bis 4 Kohlenstoffatomen im Alkylrest ist, und $Y_1$, $Y_2$ und $Y_3$ unabhängig voneinander Wasserstoff, Alkyl oder Alkoxy mit je 1 bis 12 Kohlenstoffatomen, wobei die Alkyl- und Alkoxyreste mit Hydroxyl, Methoxy oder Carbalkoxy mit 2 bis 9 Kohlenstoffatomen substituiert sein können, $—CH_2CH(OH)CH_2SO_3K$, Cyclopentyl, Cyclohexyl, Phenyl, Hydroxyl, Sulfo, Halogen, Alkyl- oder Dialkylamino mit (je) 1 bis 4 Kohlenstoffatomen im Alkylrest, Nitro, Cyano oder Carbalkoxy mit 2 bis 4 Kohlenstoffatomen sind.

11. Verbindungen nach Anspruch 10, dadurch gekennzeichnet, dass X Sauerstoff oder Schwefel ist, und $Y_1$, $Y_2$ und $Y_3$ unabhängig voneinander Wasserstoff, Alkyl oder Alkoxy mit je 1 bis 4 Kohlenstoffatomen, wobei die Alkyl- und Alkoxyreste mit Hydroxyl, Methoxy oder Carbalkoxy mit 2 bis 9 Kohlenstoffatomen substituiert sein können, Phenyl, Hydroxyl, Nitro, Cyano, $—CO_2CH_3$ oder Chlor sind.

12. Verbindungen nach Anspruch 7, dadurch gekennzeichnet, dass $R_1$ ein Heterocyclus der Formeln

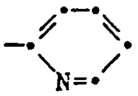 , 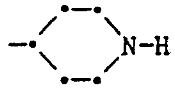 , 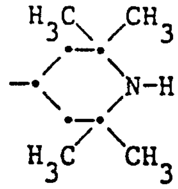 , 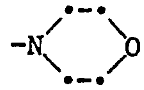 , 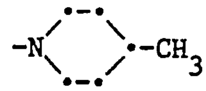 ,

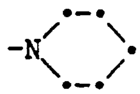 , 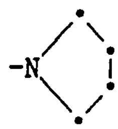 oder 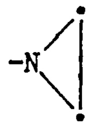 ist,

13. Verbindungen nach Anspruch 12, dadurch gekennzeichnet, dass $R_1$ ein Heterocyclus der Formeln

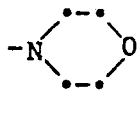 , 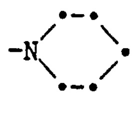 , 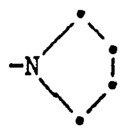 oder 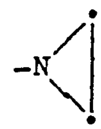 ist.

14. Verbindungen nach Anspruch 10, dadurch gekennzeichnet, dass $R_2$ und $R_3$ Reste der Formel

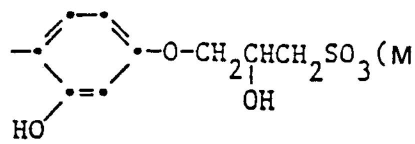

sind, worin M Natrium oder Kalium und $R_1$ Phenyl, Diphenyl, Tolyl oder p-Chlorphenyl ist.

15. Verfahren zur Herstellung der Verbindungen der Formel

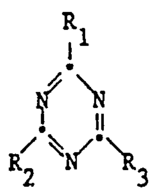 , (1)

worin $R_1$, $R_2$ und $R_3$ die in Anspruch 1 angegebene Bedeutung haben, dadurch gekennzeichnet, dass man

(I) 2,4,6-Trichlortriazin nach üblichen Methoden in mit $R_1$ oder mit $R_1$ und $R_2$ substituierte Derivate überführt,

(II) das (die) Chloratom(e) am Triazinylrest in Gegenwart von Sulfolan mit einem Resorcinderivat der Formel

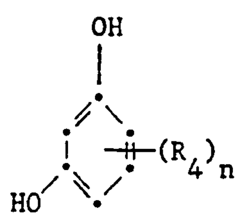 ,

worin $R_4$ und n die in Anspruch 1 angegebene Bedeutung haben, umsetzt, die erhaltenen Verbindungen isoliert und diese dann

(III) in Gegenwart einer Base mit Verbindungen der Formel

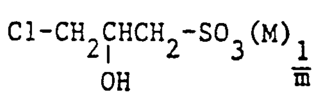 oder (3) 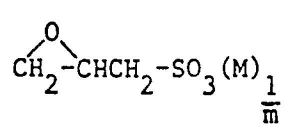 , (2)

worin M und m die in Anspruch 1 angegebene Bedeutung haben, in einem organischen Lösungsmittel

umsetzt und das Produkt isoliert.

16. Verfahren nach Anspruch 15, dadurch gekennzeichnet, dass in Stufe II ein Lösungsmittelgemisch aus Petroläther und Sulfolan im Volumenverhältnis von 0:1 bis 10:1 verwendet wird und die Reaktionstemperatur 10 bis 95°C beträgt.

17. Verfahren nach Anspruch 15, dadurch gekennzeichnet, dass in Stufe III ein Alkalimetallhydroxid oder ein Tetraalkylammoniumhydroxid als Base verwendet wird und die Reaktionstemperatur 20 bis 150°C beträgt.

18. Verfahren nach Anspruch 17, dadurch gekennzeichnet, dass die Reaktionstemperatur 20 bis 100°C beträgt.

19. Verwendung der Verbindung nach Anspruch 1 als UV-Absorber in organischen Materialien.

20. Verwendung nach Anspruch 19, dadurch gekennzeichnet, dass die organischen Materialien photographische Materialien sind.

21. Photographische Materialien, dadurch gekennzeichnet, dass sie in mindestens einer Schicht oder dem Träger eine Verbindung nach Anspruch 1 enthalten.

**Revendications**

1. Composés de formule

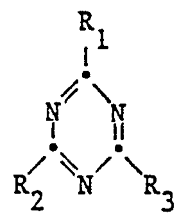

(1)

dans laquelle au moins un des substituants $R_1$, $R_2$ et $R_3$ est un résidu de formule

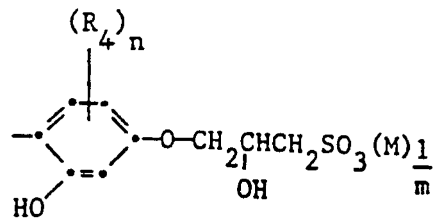

dans laquelle M est un métal alcalin ou alcalino-terreux, de l'ammonium ou du tétraalkylammonium, où les radicaux alkyle comportent indépendamment l'un de l'autre 1 à 4 atomes de carbone, m est égal à 1 ou à 2, $R_4$ est un radical alkyle ou alcoxy comportant chacun 1 à 4 atomes de carbone, un hydroxyle ou un radical de formule —COR, dans laquelle R représente un alkyle comportant 1 à 8 atomes de carbone ou un phényle et n est égal à 0, 1, 2 ou 3 et l'autre substituant (ou les autres substituants) est (ou sont) indépendamment l'un de l'autre un alkyle comportant 1 à 12 atomes de carbone, ou un alkyle comportant 1 à 12 atomes de carbone substitué par un radical méthoxy, hydroxyle, phényle, pipéridinyle, pyrrolidinyle ou carbalcoxy comportant 2 à 9 atomes de carbone, un phényle ou un naphtyle ou un phényle ou un naphtyle substitué(s) par un radical alkyle ou alcoxy comportant chacun 1 à 12 atomes de carbone, qui peuvent être substitués par un hydroxyle, un méthoxy ou un carbalcoxy comportant 2 à 9 atomes de carbone, ou $CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo, ce substituant (ou ces substituants) pouvant également être lié ou liés au résidu triazinyle par un atome d'oxygène, de soufre ou d'azote, un radical phényle étant lié par un atome d'azote au radical triazinyle pouvant en être substitué par des groupements thiazole, benzothiazole, oxazole, isoxazole et benzoxazole, ou ce substituant (ou ces substituants) étant un hétérocycle azoté comportant 3 à 6 atomes nucléaires.

2. Composés selon la revendication 1, caractérisés en ce que $R_2$ et $R_3$ sont chacun un radical de formule

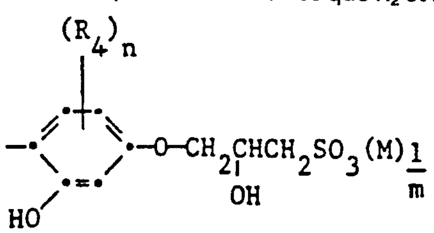

dans laquelle $R_4$ est un radical méthyle, méthoxy, hydroxyle ou acétyle, n est égal à 1, 2 ou 3, M et m possèdent la même signification que dans la revendication 1 et $R_1$ est un radical alkyle comportant 1 à 12 atomes de carbone ou un alkyle comportant 1 à 12 atomes de carbone substitué par un radical méthoxy, hydroxyle, phényle, pipéridinyle, pyrrolidinyle ou carbalcoxy comportant 2 à 9 atomes de carbone, un phényle ou un naphtyle ou un phényle ou un naphtyle substitué(s) par un radical alkyle ou alcoxy comportant chacun 1 à 12 atomes de carbone, qui peuvent être substitués par un radical hydroxyle, méthoxy ou carbalcoxy comportant 2 à 9 atomes de carbone, ou $CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo, un alkyle comportant 1 à 12 atomes de carbone lié au radical triazinyle par un atome d'oxygène, de soufre ou d'azote, ou un alkyle (lié comme indiqué ci-dessus) comportant 1 à 12 atomes de carbone substitué par un radical méthoxy, hydroxyle, phényle, pipéridinyle, pyrrolidinyle ou carbalcoxy comportant 2 à 9 atomes de carbone, un phényle ou un naphtyle (liés comme indiqué ci-dessus) ou un phényle ou un naphtyle (liés comme indiqué ci-dessus) substitué(s) par un radical alkyle ou alcoxy comportant chacun 1 à 12 atomes de carbone, qui peuvent être substitués par un radical hydroxyle, méthoxy ou carbalcoxy comportant 2 à 9 atomes de carbones, ou $CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo, ou un hétérocycle azoté comportant 3 à 6 atomes nucléaires.

3. Composés selon la revendication 2, caractérisés en ce que $R_1$ est un phényle ou un naphtyle ou un phényle ou un naphtyle substitué(s) par un radical alkyle ou alcoxy comportant chacun 1 à 12 atomes de carbone, qui peuvent être substitués par un radical hydroxyle, méthoxy ou carbalcoxy comportant 2 à 9 atomes de carbones, ou $CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo, ou un phényle ou un naphtyle lié à un résidu triazinyle par un atome d'oxygène, de soufre ou d'azote ou un phényle ou un naphtyle (liés comme indiqué ci-dessus) substitué(s) par un radical alkyle ou alcoxy comportant chacun 1 à 12 atomes de carbone, qui peuvent être substitués par un radical hydroxyle, méthoxy ou carbalcoxy comportant 2 à 9 atomes de carbones, ou $CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo.

4. Composés selon la revendication 3, caractérisés en ce que $R_1$ est un radical phényle, anilino, ou N-alkyl-anilino, ces radicaux pouvant être substitués par un radical alkyle ou alcoxy comportant chacun 1 à 12 atomes de carbone, un radical phényle, hydroxyle, halogéno, nitro, cyano ou carbalcoxy comportant 2 à 9 atomes de carbone, des groupements thiazole, benzothiazole, oxazole, isoxazole ou benzoxazole.

5. Composés selon la revendication 4, caractérisés en ce que $R_1$ est un phényle ou un résidu de formule

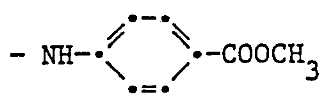

6. Composés selon la revendication 1, caractérisés en ce que $R_2$ et $R_3$ sont des résidus de formule

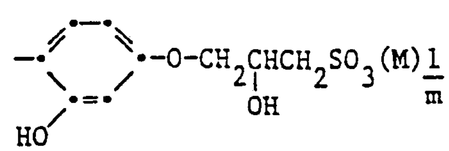

M et m possèdant la signification indiquée à la revendication 1, $R_1$ possède la même signification que $R_2$ et $R_3$ ou $R_1$ est un radical alkyle comportant 1 à 12 atomes de carbone ou un radical alkyle comportant 1 à 12 atomes de carbone, substitué par un radical méthoxy, hydroxyle, phényle, pipéridinyle, pyrrolidinyle ou carbalcoxy comportant 2 à 9 atomes de carbone, un phényle ou un naphtyle ou un phényle ou un naphtyle substitué(s) par un radical alkyle ou alcoxy comportant chacun 1 à 12 atomes de carbone, qui peuvent être substitués par un radical hydroxyle, méthoxy ou carbalcoxy comportant 2 à 9 atomes de carbone, ou —$CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo, un radical alkyle comportant 1 à 12 atomes de carbone lié au radical triazinyle par un atome d'oxygène, de soufre ou d'azote, ou un radical alkyle comportant 1 à 12 atomes de carbone (lié comme indiqué ci-dessus) substitué par un radical méthoxy, hydroxyle, phényle, pipéridinyle, pyrrolidinyle ou carbalcoxy comportant 2 à 9 atomes de carbone, un phényle ou un naphtyle (liés comme indiqué ci-dessus) ou un phényle ou un naphtyle (liés comme indiqué ci-dessus) substitué(s) par un radical alkyle ou alcoxy comportant chacun 1 à 12 atomes de carbone, qui peuvent être substitués par un radical hydroxyle, méthoxy ou carbalcoxy comportant 2 à 9 atomes de

carbone, ou $CH_2CH(OH)CH_2SO_3K$, un radical cyclopentyle, cyclohexyle, phényle, hydroxyle, halogéno, (di)alkylamino comportant (chacun) 1 à 4 atomes de carbone dans le radical alkyle, un groupe nitro, cyano, carbalcoxy comportant 2 à 9 atomes de carbone ou des groupements sulfo, ou un hétérocycle azoté comportant 3 à 6 noyaux nucléaires.

7. Composés selon la revendication 6, caractérisés en ce que $R_2$ et $R_3$ sont chacun un résidu de formule

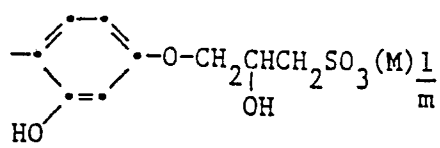

M représente du sodium, du potassium, du calcium, du magnésium, de l'ammonium ou du tétraalkylammonium comportant chacun 1 à 4 atomes de carbone dans les radicaux alkyle et m est égal à 1 ou à 2, $R_1$ possède la même valeur que $R_2$ et $R_3$ ou $R_1$ est un alkyle comportant 1 à 12 atomes de carbone, un phényle, un alkyle comportant 1 à 12 atomes de carbone lié au radical triazinyle par de l'oxygène, du soufre ou un radical imino- ou alkylimino comportant 1 à 4 atomes de carbone dans le radical alkyle ou un phényle (lié comme indiquée ci-dessus) ou un hétérocycle azoté comportant 3 à 6 noyaux nucléaires.

8. Composés selon la revendication 7, caractérisés en ce que $R_2$ et $R_3$ possèdent la signification indiquée dans la revendication 7 et en ce que $R_1$ possède la même valeur que $R_2$ et $R_3$ ou en ce que $R_1$ est un radical alkyle, alcoxy, alkylamino ou thioalkyle comportant chacun 1 à 12 atomes de carbone, ces résidus pouvant être substitués par un radical hydroxyle, phényle, méthoxy, pipéridinyle, pyrrolidinyle ou carbalcoxy comportant 2 à 9 atomes de carbone.

9. Composés selon la revendication 8, caractérisés en ce que $R_1$ est un radical alkyle ou alcoxy comportant chacun 1 à 4 atomes de carbone, un radical benzylamino ou thioalkyle comportant 8 à 12 atomes de carbone.

10. Composés selon la revendication 7, caractérisés en que $R_1$ est un radical de formule

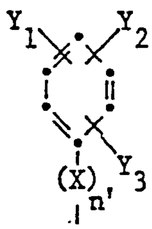

dans laquelle n' est égal à 0 ou à 1, X correspond à de l'oxygène, à du souffre, à un radical imino ou alkylimino comportant 1 à 4 atomes de carbone dans le radical alkyle et en ce que $Y_1$, $Y_2$ et $Y_3$ peuvent représenter indépendamment l'un de l'autre de l'hydrogène, un radical alkyle ou alcoxy comportant chacun 1 à 12 atomes de carbone, les radicaux alkyle et alcoxy pouvant être substitués par un radical hydroxyle, méthoxy ou carbalcoxy comportant 2 à 9 atomes de carbone, $—CH_2CH(OH)CH_2SO_3K$, un radical cyclopentyle, cyclohexyle, phényle, hydroxyle, sulfo, halogéno, ou (di)alkylamino comportant (chacun) 1 à 4 atomes de carbone dans le radical alkyle, un groupe nitro, cyano ou carbalcoxy comportant 2 à 4 atomes de carbone.

11. Composés selon la revendication 10, caractérisé en ce que X correspond à de l'oxygène ou à du soufre et en ce que $Y_1$, $Y_2$ et $Y_3$ peuvent représenter indépendamment l'un de l'autre de l'hydrogène, un radical alkyle ou alcoxy comportant chacun 1 à 4 atomes de carbone, les radicaux alkyle et alcoxy pouvant être substitués par un radical hydroxyle, méthoxy ou carbalcoxy comportant 2 à 9 atomes de carbone, un radical phényle, hydroxyle, nitro, cyano, $—CO_2CH_3$ ou du chlore.

12. Composés selon la revendication 7, caractérisés en ce que R est un hétérocycle de formules

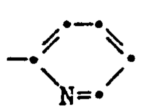 , 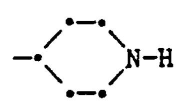 , 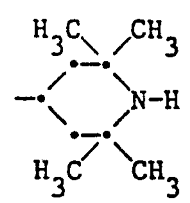 , 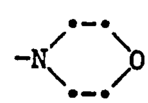, 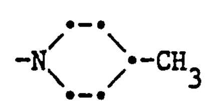 ,

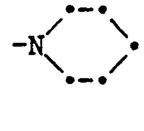 , 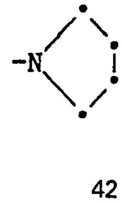 ou 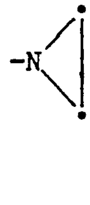 ,

13. Composés selon la revendication 12, caractérisés en ce que $R_1$ est un hétérocycle de formules

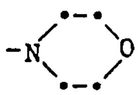 , 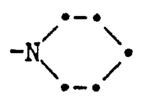 , 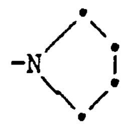 ou 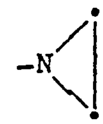

14. Composés selon la revendication 10, caractérisés en ce que $R_2$ et $R_3$ sont des radicaux de formule

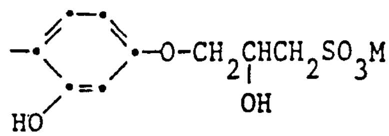

dans laquelle M est du sodium ou du potassium et $R_1$ est un radical phényle, diphényle, tolyle ou p-chlorophényle.

15. Procédé de fabrication de composés de formule

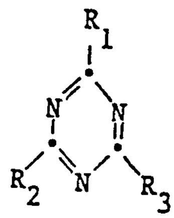

(1)

dans laquelle $R_1$, $R_2$ et $R_3$ possèdent la signification indiquée dans la revendication 1, caractérisé en ce que l'on

(I) convertit la 2,4,6-trichlorotriazine selon des méthodes usuelles en dérivés substitués par $R_1$ ou par $R_1$ et $R_2$,

(II) fait réagir le ou les atomes de chlore lié(s) au radical triazinyle en présence de Sulfolane avec un dérivé de résorcine de formule

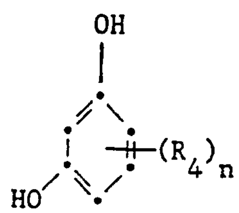 ,

dans laquelle $R_4$ et n possèdent la valeur indiquée dans la revendication 1, en ce que l'on isole les composés obtenus, en ce que l'on fait réagir ensuite ceux-ci dans un solvant organique

(III) en présence d'une base avec des composés de formule

(2) 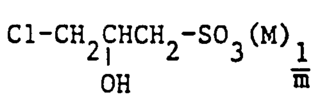 ou (3) 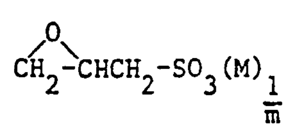 ,

dans laquelle M et m possèdent la signification indiquée à la revendication 1, et en ce que l'on isole le produit.

16. Procédé selon la revendication 15, caractérisé en ce que l'on met en oeuvre à l'étape II un mélange de solvants constitué d'éther de pétrole et de Sulfolane dans des proportions volumiques allant de 0:1 à 10:1 et en ce que la température de la réaction atteint 10 à 95°C.

17. Procédé selon la revendication 15, caractérisé en ce que l'on met en oeuvre à l'étape III un hydroxyde de métal alcalin ou un hydroxyde de tétraalkylammonium comme base, et en ce que la température de la réaction atteint 20 à 150°C.

18. Procédé selon la revendication 17, caractérisé en ce que la température de la réaction atteint 20 à 100 °C.

19. Mise en oeuvre des composés selon la revendication 1, comme absorbeur d'UV dans des matériaux organiques.

20. Mise en oeuvre selon la revendication 19, caractérisée en ce que lesdits matériaux organiques sont des matériaux photographiques.

21. Matériaux photographiques, caractérisés en ce qu'ils renferment un composé selon la revendication 1 dans au moins une couche ou dans le support.

**Claims**

1. Compounds of the formula

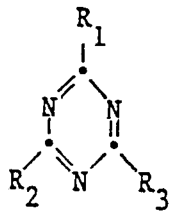

(1)

in which at least one of the substituents $R_1$, $R_2$ and $R_3$ is a radical of the formula

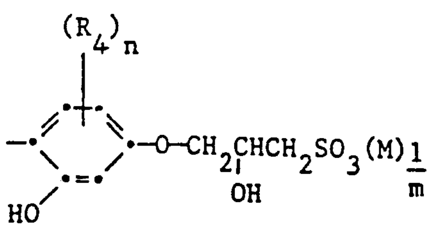

in which M is an alkali or alkaline earth metal, ammonium or tetraalkylammonium, in which the alkyl radicals independently of one another contain 1 to 4 carbon atoms, m is 1 or 2, $R_4$ is alkyl or alkoxy each having 1 to 4 carbon atoms, hydroxyl or a radical of the formula —COR, in which R is alkyl having 1 to 8 carbon atoms or phenyl, and n is 0, 1, 2 or 3, and the remaining substituent or substituents is or are independently of one another alkyl having 1 to 12 carbon atoms or alkyl having 1 to 12 carbon atoms which is substituted by methoxy, hydroxyl, phenyl, piperidinyl, pyrrolidinyl or carbalkoxy having 2 to 9 carbon atoms, is or are phenyl or naphthyl or phenyl or naphthyl which are substituted by alkyl or alkoxy each having 1 to 12 carbon atoms, which can be substituted by hydroxyl, methoxy or carbalkoxy having 2 to 9 carbon atoms, or by $—CH_2CH(OH)CH_2SO_3K$, cyclopentyl, cyclohexyl, phenyl, hydroxyl, halogen, (di-)alkylamino (each) having 1 to 4 carbon atoms in the alkyl radical, nitro, cyano or carbalkoxy having 2 to 9 carbon atoms or sulfo groups, it being possible for this substituent or these substituents also to be bonded to the triazinyl radical via an oxygen, sulphur or nitrogen atom, it being possible for a phenyl radical bonded to the triazinyl ring via a nitrogen atom to be additionally substituted by thiazole, benzothiazole, oxazole, isoxazole or benzoxazole groups, or this substituent or these substituents being a nitrogen-containing heterocyclic ring having 3 to 6 ring atoms.

2. Compounds according to Claim 1, characterized in that $R_2$ and $R_3$ are a radical of the formula

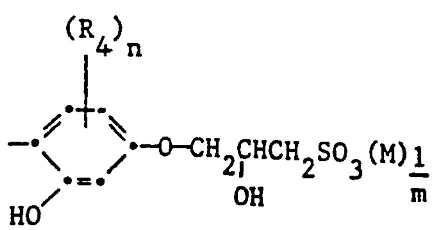

in which $R_4$ is methyl, methoxy, hydroxyl or acetyl, n is 1, 2 or 3, M and m are as defined in Claim 1 and $R_1$ is alkyl having 1 to 12 carbon atoms or alkyl having 1 to 12 carbon atoms which is substituted by methoxy, hydroxyl, phenyl, piperidinyl, pyrrolidinyl or carbalkoxy having 2 to 9 carbon atoms, is phenyl or naphthyl or phenyl or naphthyl which are substituted by alkyl or alkoxy each having 1 to 12 carbon atoms, which can be substituted by hydroxyl, methoxy or carbalkoxy having 2 to 9 carbon atoms, or by $—CH_2CH(OH)CH_2SO_3K$, cyclopentyl, cyclohexyl, phenyl, hydroxyl, halogen, (di-)alkylamino (each) having 1

to 4 carbon atoms in the alkyl radical, nitro, cyano or carbalkoxy having 2 to 9 carbon atoms or sulfo groups, or is alkyl having 1 to 12 carbon atoms which is bonded to the triazinyl radical via an oxygen, sulphur or nitrogen atom or alkyl having 1 to 12 carbon atoms, which is substituted by methoxy, hydroxyl, phenyl, piperidinyl, pyrrolidinyl or carbalkoxy having 2 to 9 carbon atoms, or is phenyl or naphthyl or phenyl or naphthyl which are substituted by alkyl or alkoxy each having 1 to 12 carbon atoms, which can be substituted by hydroxyl, methoxy or carbalkoxy having 2 to 9 carbon atoms, or by — $CH_2CH(OH)CH_2SO_3K$, cyclopentyl, cyclohexyl, phenyl, hydroxyl, halogen, (di-)alkylamino (each) having 1 to 4 carbon atoms in the alkyl radical, nitro, cyano or carbalkoxy having 2 to 9 carbon atoms or sulfo groups, or is a nitrogen-containing heterocyclic ring having 3 to 6 ring atoms.

3. Compounds according to Claim 2, characterized in that $R_1$ is phenyl or naphthyl or phenyl or naphthyl which are substituted by alkyl or alkoxy each having 1 to 12 carbon atoms, which can be substituted by hydroxyl, methoxy or carbalkoxy having 2 to 9 carbon atoms, or by —$CH_2CH(OH)CH_2SO_3K$, cyclopentyl, cyclohexyl, phenyl, hydroxyl, halogen, (di-)alkylamino (each) having 1 to 4 carbon atoms in the alkyl radical, nitro, cyano or carbalkoxy having 2 to 9 carbon atoms or sulfo groups, or is phenyl or naphthyl bonded to the triazinyl radical via an oxygen, sulphur or nitrogen atom or is phenyl or naphthyl which are substituted by alkyl or alkoxy each having 1 to 12 carbon atoms, which can be substituted by hydroxyl, methoxy or carbalkoxy having 2 to 9 carbon atoms, or by —$CH_2CH(OH)CH_2SO_3K$, cyclopentyl, cyclohexyl, phenyl, hydroxyl, halogen, (di-)alkylamino (each) having 1 to 4 carbon atoms in the alkyl radical, nitro, cyano or carbalkoxy having 2 to 9 carbon atoms or sulfo groups.

4. Compounds according to Claim 3, characterized in that $R_1$ is phenyl, anilino or N-alkylanilino, these radicals being unsubstituted or substituted by alkyl or alkoxy each having 1 to 12 carbon atoms, phenyl, hydroxyl, halogen, nitro, cyano or carbalkoxy having 2 to 9 carbon atoms or thiazole, benzothiazole, oxazole, isoxazole or benzoxazole groups.

5. Compounds according to Claim 4, characterized in that $R_1$ is phenyl or a radical of the formula

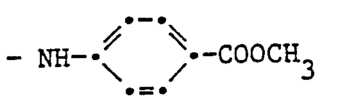 .

6. Compounds according to Claim 1, wherein $R_2$ and $R_3$ are radicals of the formula

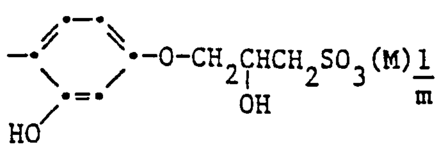

M and m are as defined in Claim 1, $R_1$ is as defined for $R_2$ and $R_3$ or $R_1$ is alkyl having 1 to 12 carbon atoms or alkyl having 1 to 12 carbon atoms which is substituted by methoxy, hydroxyl, phenyl, piperidinyl, pyrrolidinyl or carbalkoxy having 2 to 9 carbon atoms, is phenyl or naphthyl or phenyl or naphthyl which by alkyl or alkoxy each having 1 to 12 carbon atoms, which can be substituted by hydroxyl, methoxy or carbalkoxy having 2 to 9 carbon atoms, or by —$CH_2CH(OH)CH_2SO_3K$, cyclopentyl, cyclohexyl, phenyl, hydroxyl, halogen, (di-)alkylamino (each) having 1 to 4 carbon atoms in the alkyl radical, nitro, cyano or carbalkoxy having 2 to 9 carbon atoms or sulfo groups, or is alkyl having 1 to 12 carbon atoms which is bonded to the triazinyl radical via an oxygen, sulphur or nitrogen atom or alkyl having 1 to 12 carbon atoms, which is substituted by methoxy, hydroxyl, phenyl, piperidinyl, pyrrolidinyl or carbalkoxy having 2 to 9 carbon atoms, or is phenyl or naphthyl or phenyl or naphthyl which are substituted by alkyl or alkoxy each having 1 to 12 carbon atoms, which can be substituted by hydroxyl, methoxy or carbalkoxy having 2 to 9 carbon atoms, or by —$CH_2CH(OH)CH_2SO_3K$, cyclopentyl, cyclohexyl, phenyl, hydroxyl, halogen, (di-)alkylamino (each) having 1 to 4 carbon atoms in the alkyl radical, nitro, cyano or carbalkoxy having 2 to 9 carbon atoms or sulfo groups, or is a nitrogen-containing heterocyclic ring having 3 to 6 ring atoms.

7. Compounds according to Claim 6, characterized in that $R_2$ and $R_3$ are a radical of the formula

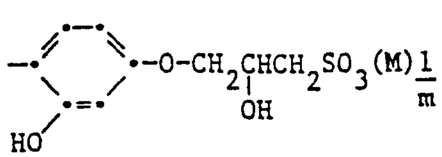 ,

M is sodium, potassium, calcium, magnesium, ammonium or tetraalkylammonium having 1 to 4 carbon atoms in each of the alkyl radicals and m is 1 or 2, and $R_1$ is as defined for $R_2$ and $R_3$ or $R_1$ is alkyl having 1 to 12 carbon atoms, phenyl, or alkyl having 1 to 12 carbon atoms or phenyl which are bondeed to the triazinyl radical via oxygen, sulphur or imino or alkylamino having 1 to 4 carbon atoms in the alkyl radical, or is a nitrogen-containing heterocyclic ring having 3 to 6 ring atoms.

8. Compounds according to Claim 7, characterized in that $R_2$ and $R_3$ are as defined in Claim 7 and $R_1$ is as defined for $R_2$ and $R_3$ or $R_1$ is alkyl, alkoxy, alkylamino or thioalkyl each having 1 to 12 carbon atoms, these radicals being unsubstituted or substituted by hydroxyl, phenyl, methoxy, piperidinyl, pyrrolidinyl or carbalkoxy having 2 to 9 carbon atoms.

9. Compounds according to Claim 8, characterized in that $R_1$ is alkyl or alkoxy each having 1 to 4 carbon atoms, benzylamino or thioalkyl having 8 to 12 carbon atoms.

10. Compounds according to Claim 7, characterized in that $R_1$ is a radical of the formula

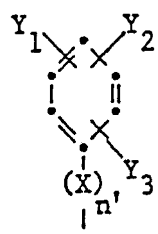

in which n' is 0 or 1, X is oxygen, sulphur, imino or alkylimino having 1 to 4 carbon atoms in the alkyl radical, and $Y_1$, $Y_2$ and $Y_3$ independently of one another are hydrogen or alkyl or alkoxy each having 1 to 12 carbon atoms, the alkyl and alkoxy radicals being unsubstituted or substituted by hydroxyl, methoxy or carbalkoxy having 2 to 9 carbon atoms, or are $-CH_2CH(OH)CH_2SO_3K$, cyclopentyl, cyclohexyl, phenyl, hydroxyl, sulfo, halogen, alkyl- or dialkylamino having 1 to 4 carbon atoms in (each of) the alkyl radical(s), nitro, cyano or carbalkoxy having 2 to 4 carbon atoms.

11. Compounds according to Claim 10, characterized in that X is oxygen or sulphur, and $Y_1$, $Y_2$ and $Y_3$ independently of one another are hydrogen or alkyl or alkoxy each having 1 to 4 carbon atoms, the alkyl and alkoxy radicals being unsubstituted or substituted by hydroxyl, methoxy or carbalkoxy having 2 to 9 carbon atoms, or are phenyl, hydroxyl, nitro, cyano $-CO_2CH_3$ or chlorine.

12. Compounds according to Claim 7, characterized in that $R_1$ is a heterocyclic ring of the formulae

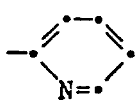 , 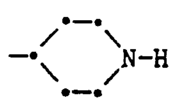 , 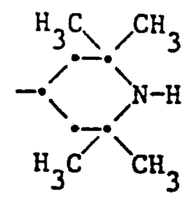 , 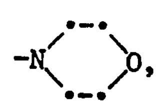, 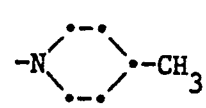 ,

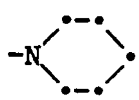 , 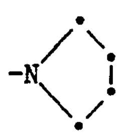 or 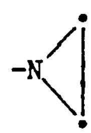 ,

13. Compounds according to Claim 12, characterized in that $R_1$ is a heterocyclic ring of the formulae

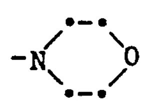 , 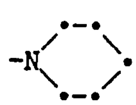 , 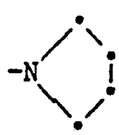 or 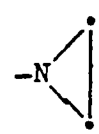

14. Compounds according to Claim 10, characterized in that $R_2$ and $R_3$ are radicals of the formula

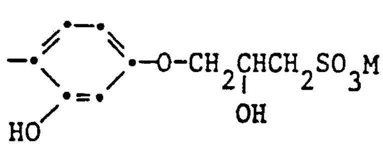

in which M is sodium or potassium and $R_1$ is phenyl, diphenyl, tolyl or p-chlorophenyl.

15. Process for the preparation of compounds of the formula

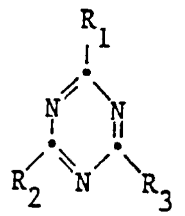

(1)

in which $R_1$, $R_2$ and $R_3$ are as defined in Claim 1, characterized in that
(I) 2,4,6-triochlorotriazine is converted by conventional methods into derivatives substituted by $R_1$ or by $R_1$ and $R_2$,
(II) the chlorine atom(s) on the triazinyl radical is or are reacted in the presence of sulfolan with a resorcinol derivative of the formula

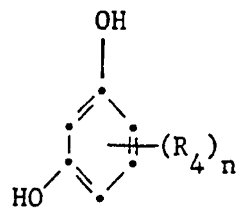

,

in which $R_4$ and n are as defined in Claim 1, the resulting compounds are isolated and then reacted (III) in the presence of a base with compounds of the formula

(2) 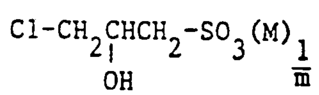 or (3) 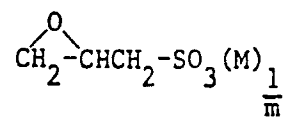 ,

in which M and m are as defined in Claim 1, in an organic solvent and the product is isolated.

16. Process according to Claim 15, characterized in that in stage II a solvent mixture of petroleum ether and sulfolan in a ratio of 0:1 to 10:1 by volume is used and the reaction temperature is 10 to 95°C.

17. Process according to Claim 15, characterized in that in stage III an alkali metal hydroxide or a tetraalkylammonium hydroxide is used as the base and the reaction temperature is 20 to 150°C.

18. Process according to Claim 17, characterized in that the reaction temperature is 20 to 100°C.

19. Use of compounds according to Claim 1 as UV absorbers in organic materials.

20. Use according to Claim 19, characterized in that the organic materials are photographic materials.

21. Photographic materials, characterized in that they contain, in at least one layer or the base, a compound according to Claim 1.